# NEW EUROPEAN PATENT SPECIFICATION

(11) **EP 0 519 001 B2**
(45) Date of publication and mention of the opposition decision: **19.12.2007**
(45) Mention of the grant of the patent: 31.10.2001
(21) Application number: 91906615.9
(22) Date of filing: 08.03.1991
(51) Int. Cl.: C07K 1/00, A61K 39/21, C12N 15/49

(54) **PURIFIED gp120 COMPOSITION RETAINING NATURAL CONFORMATION**
GEREINIGTES GP120, IN DEM SEINE NATÜRLICHE KONFORMATION ERHALTEN BLEIBT
COMPOSITION DE gp120 PURIFIEE PRESERVANT SA CONFORMATION NATURELLE

(30) Priority: 09.03.1990 US 490858
(43) Date of publication of application: 23.12.1992
(73) Proprietor: Novartis Vaccines and Diagnostics, Inc., Emeryville, CA 94608 (US)
(72) Inventor: HAIGWOOD, Nancy, L., Oakland, CA 94611 (US); SCANDELLA, Carl, J., Oakland, CA 94611 (US)
(74) Representative: Hallybone, Huw George
(86) International application number: PCT/US1991/001484
(87) International publication number: WO 1991/013906

(56) References cited:
- EP-A- 0 315 459
- LASKY L A ET AL: "Delineation of a region of the human immunodeficiency virus type 1 gp120 glycoprotein critical for interaction with the CD4 receptor." CELL, vol. 50, 11 September 1987 (1987-09-11), pages 976-985, XP001189314 Cell press
- SCHNITTMAN S M ET AL: "Characterization of GP120 binding to CD4 and an assay that measures ability of sera to inhibit this binding." THE JOURNAL OF IMMUNOLOGY, vol. 141, no. 12, 15 December 1988 (1988-12-15), pages 4181-4186, XP001189309 The american association of immunologists, US ISBN: 0022-1767
- ROBEY ET AL: "Prospect for prevention of human immunodeficiency virus infection: purified 120-kDa envelope glycoprotei induces neutralizing antibody" PROCEEDINGS OF THE NATIONAL ACADEMY OF SCIENCES OF USA, NATIONAL ACADEMY OF SCIENCE. WASHINGTON, US, vol. 83, no. 18, September 1986 (1986-09), pages 7023-7027, XP002129495 ISSN: 0027-8424
- BERMAN P W ET AL: "HUMAN IMMUNODEFICIENCY VIRUS TYPE 1 CHALLENGE OF CHIMPANZEES IMMUNIZED WITH RECOMBINANT ENVELOPE GLYCOPROTEIN GP120" PROCEEDINGS OF THE NATIONAL ACADEMY OF SCIENCES OF USA, NATIONAL ACADEMY OF SCIENCE. WASHINGTON, US, vol. 85, 1 July 1988 (1988-07-01), pages 5200-5204, XP002060891 ISSN: 0027-8424

## Description

### Technical Field

The present invention is directed generally to the field of protein purification and more particularly to the purification of full-length non-fusion glycoprotein gp120 from HIV-SF2.

### Background

Attempts at making vaccines against HIV-1 have met with limited success, as measured by the criterion of achieving in animals an immune response similar or equivalent to that of humans that are sero positive to HIV-1. The major goal, not previously attained, has been the generation of antibodies that are virus neutralizing in vitro at titers reaching both the level and complexity (i.e., ability to neutralize more than one isolate) seen in human sera from infected individuals. All of the neutralizing antibodies in humans have mapped to the envelope protein, gp160, or one of its component parts (gp120 or gp41), and thus most vaccine efforts have concentrated on the development of envelope-protein-related antigens.

Five types of such antigens have been developed: (1) purified gp120 derived from HIV-infected tissue culture cells (referred herein as "viral-derived gp120"); (2) gp120 made in cells infected with recombinant viruses, such as vaccinia or baculovirus ("live-virus-vector-derived gp120 and gp160"); (3) recombinant gp120 made in mammalian cells ("recombinant mammalian gp120," sometimes referred to incorrectly as recombinant native gp120); (4) recombinant denatured polypeptides that represent all or various portions of gp120 and gp41 ("recombinant denatured antigens"); and (5) peptides that represent small segments of gp120 and gp41 ("peptides").

Immunogenicity experiments have been completed with all of these types of antigens, with fairly uniform results. In general, the antigens are highly immunogenic as adjuvanted in a variety of species. They have generated antibodies capable of neutralizing the homologous isolate of HIV-1, but they poorly or not at all neutralize non-homologous isolates. The levels of neutralization also have not (in general) reached the level of neutralizing titer found in infected humans.

For example, fully glycosylated gp120 natural purified from virus or produced by genetically engineered mammalian cells, non-glycosylated gp120 produced in yeast, and a fragment of gp120 produced in E. coli can all elicit HIV-1 neutralizing antibodies in experimental animals. For the most part, the responses of animals immunized with virion or recombinant gp120 antigens are effective in neutralizing only the virus isolate from which the gp120 antigen originated. One exception is the work of Berman et al. (reference 1 below) showing that purified recombinant HIV-1 gp120 secreted by genetically engineered Chinese hamster ovary cells elicited group-specific neutralizing antibodies in chimpanzees.

Another factor that has been particularly difficult to overcome when preparing HIV-1 vaccines is sequence diversity. HIV-1 and HIV-2 are characterized by having a very high level of sequence diversity that is most pronounced in the gp120 portion of the envelope. This sequence diversity is clustered in regions known as hypervariable regions. Many groups have proposed using a vaccine cocktail, comprising antigenic substances derived from a variety of HIV isolates, to provide protection against a broad range of infective sources.

Accordingly, there remains a need for an antigenic substance having immunological and other protein/protein binding properties of gp120 as it is presented on an HIV-1 virus particle. In particular, antigenic substances capable of inducing neutralizing antibodies, preferably using a single source material that induces neutralizing antibodies against a variety of field isolates, are highly desirable.

### Relevant Literature

The following publications are all directed to the five types of vaccine candidates described above:
(1) Berman et al., "Human Immunodeficiency Virus Type I Challenge of Chimpanzees Immunized with Recombinant Envelope Glycoprotein gp120," Proc. Natl. Acad. Sci. USA (1988) 85: 5200-5204;
(2) Berman et al., "Expression and Immunogenicity of the Extracellular Domain of the Human Immunodeficiency Virus Type I Envelope Glycoprotein, gp160," Journal of Virology (1989) 63: 3489-3498;
(3) Nara et al., "Purified Envelope Glycoproteins from Human Immunodeficiency Virus Type I Variance Induced Individual, Type-Specific Neutralizing Antibodies," Journal of Virology (1988) 62: 2622-2628;
(4) Arthur et al., "Serological Responses in Chimpanzees Inoculated with Human Immunodeficiency Virus Glycoprotein (gp120) Subunit Vaccine," Proc. Natl. Acad. Sci. USA (1987) 84: 8583-8587;
(5) Evans et al., "An Engineered Polio Virus Chimaera Elicits Broadly Reactive HIV-1 Neutralizing Antibodies," Nature (1989) 339: 385-388;
(6) Barrett et al., "Large-Scale Production and Purification of a Vaccinia Recombinant-Derived HIV-1 gp160 and Analysis of its Immunogenicity," AIDS Research and Human Retroviruses (1989) 5: 159-171;
(7) Earl et al., "Isolate- and Group-Specific Immune Response to the Envelope Protein of Human Immunodeficiency Virus Induced by a Live Recombinant Vaccinia Virus in Macaques," AIDS Research and Human Retroviruses (1989) 5: 23-32;
(8) Putney et al., "HTLV-III/LAV-Neutralizing Antibodies to an E. coli-produced Fragment of the Virus Envelope," Science (1986) 234: 1392-1395;
(9) Steimer et al., "Genetically Engineered Human Immunodeficiency Envelope Glycoprotein gp120 Produced in Yeast is the Target of Neutralizing Antibodies," Vaccines 87 (1987) 236-241;
(10) Steimer et al., "Recombinant env and gag Polypeptides in Characterizing HIV-1-Neutralizing Antibodies," Vaccines 88 (1988) 347-355;
(11) Ho et al., "Human Immunodeficiency Virus Neutralizing Antibodies Recognize Several Conserved Domains on the Envelope Glycoproteins," Journal of Virology (1987) 61: 2024-2028; and
(12) Palker et al., "Type-Specific Neutralization of the Human Immunodeficiency Virus with Antibodies to env-Encoded Synthetic Peptides," Proc. Natl. Acad. Sci. USA (1988) 85: 1932-1936.

### SUMMARY OF THE INVENTION

The invention provides the process of claim 1.

### DESCRIPTION OF THE DRAWINGS

The invention will be better understood by reference to the following description of specific embodiments in combination with the drawings that are part of the present specification, wherein:

Figure 1 is a schematic diagram of an exemplary expression plasmid for the production of recombinant HIV-1 gp120 (rgp120).

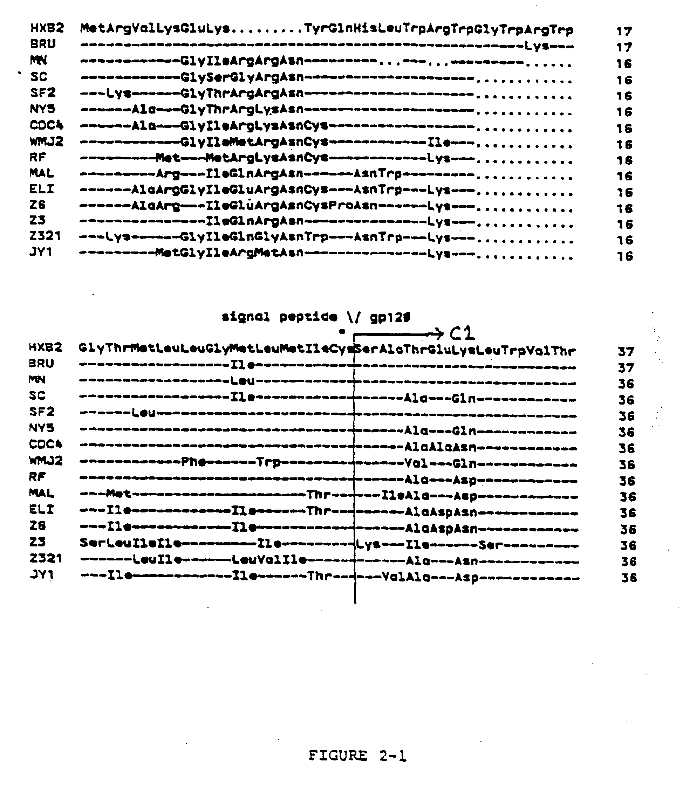

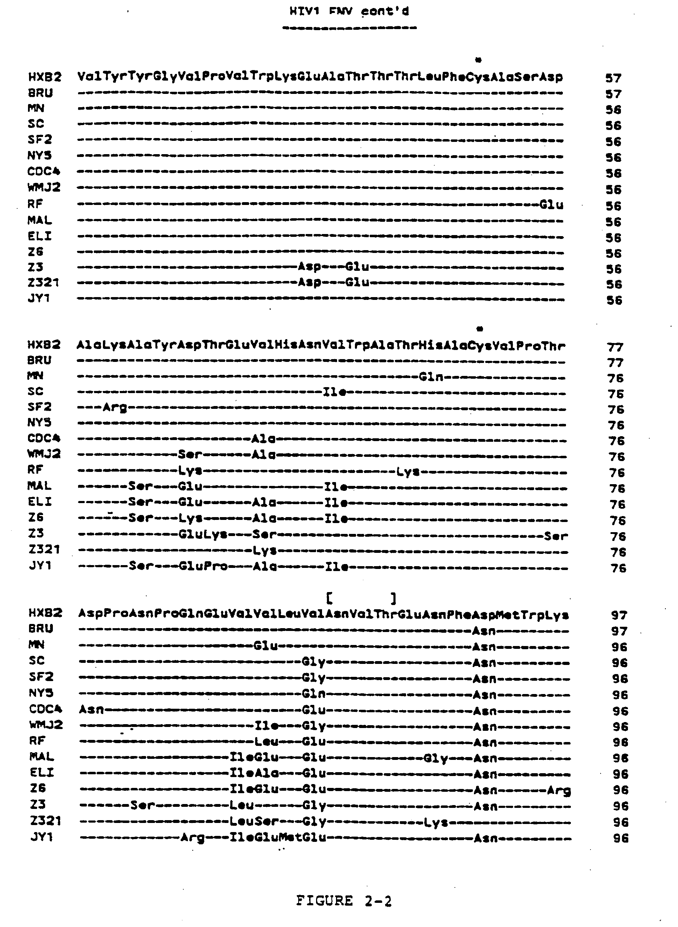

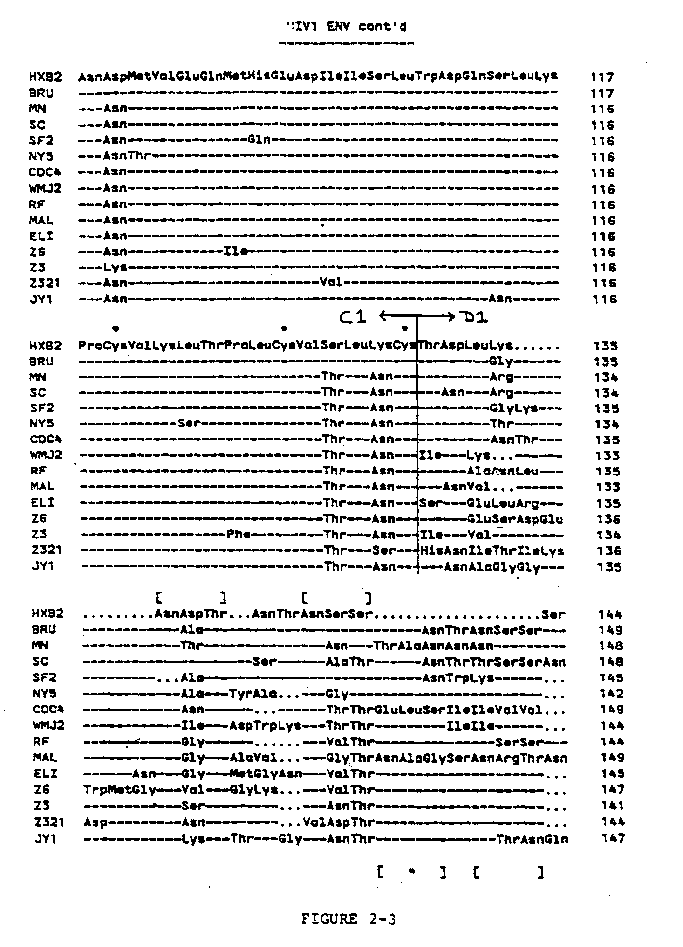

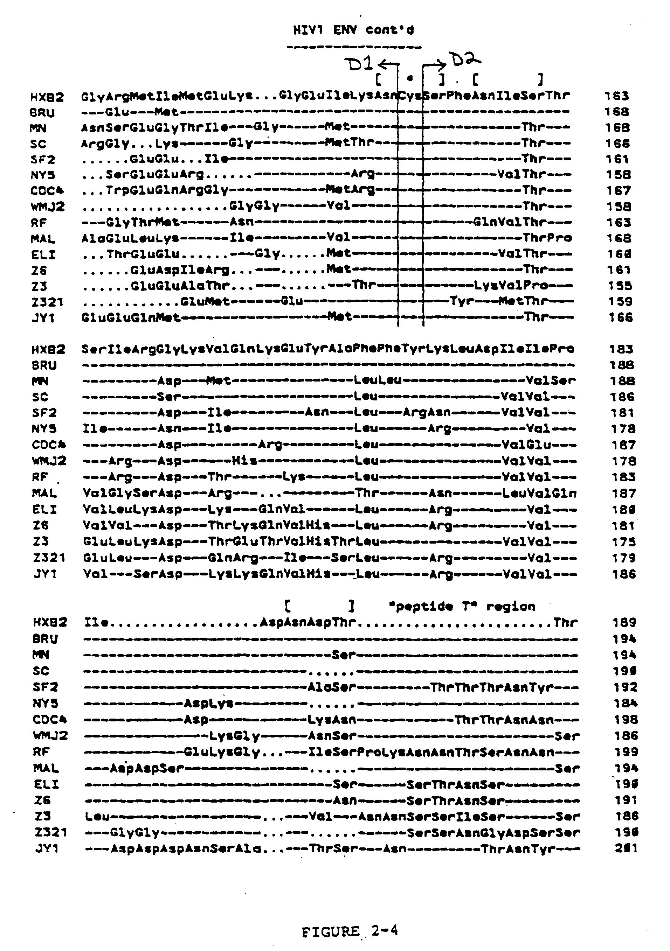

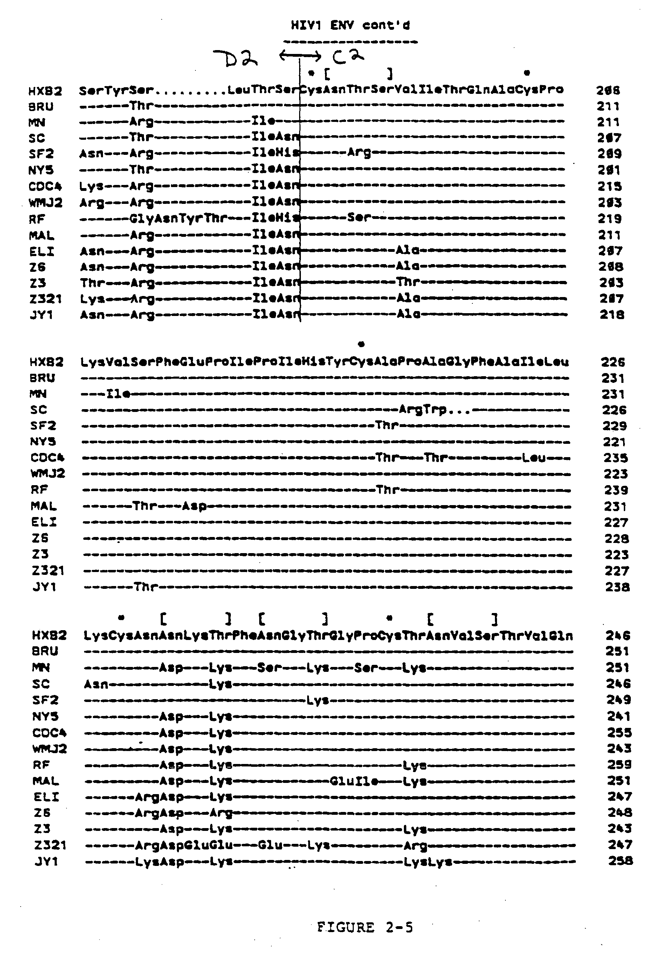

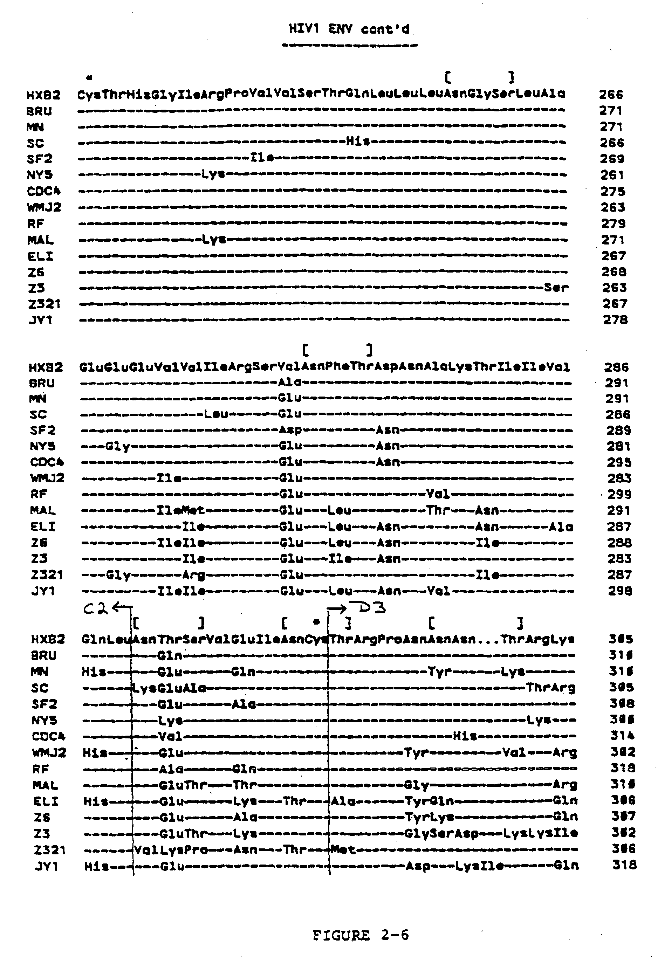

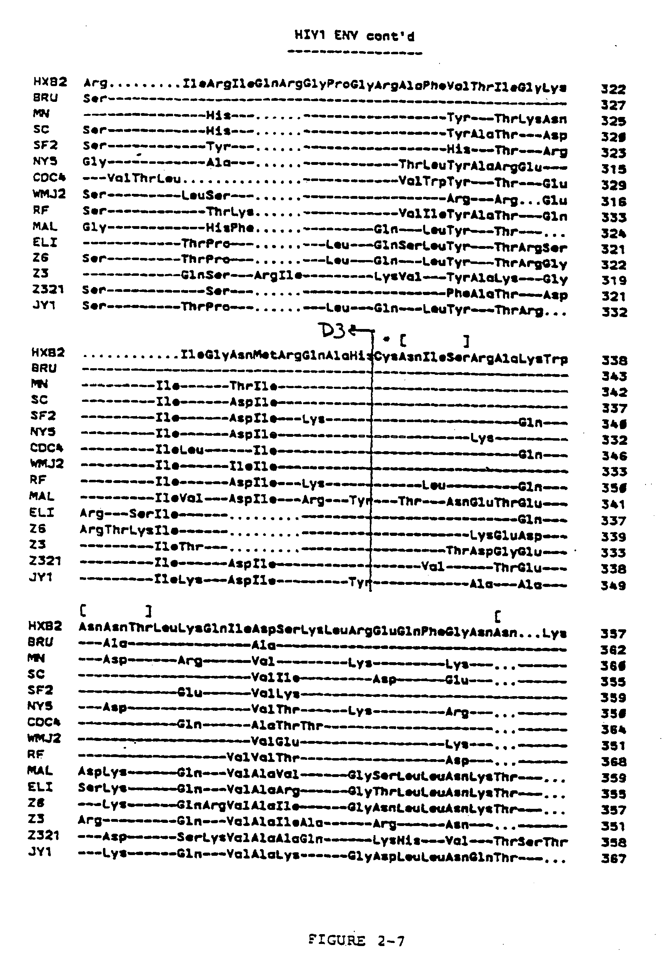

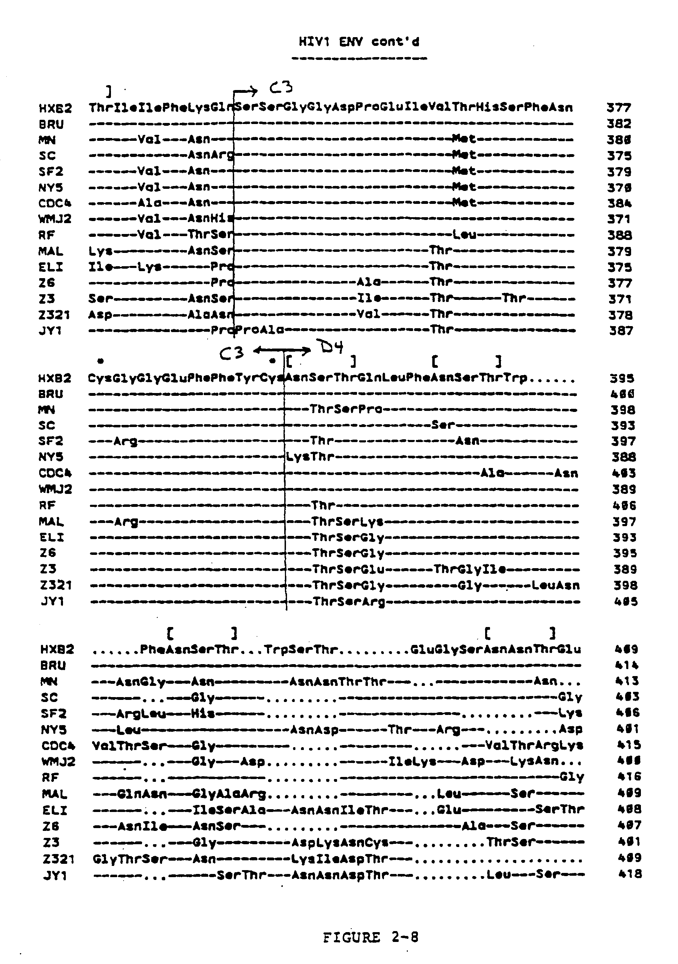

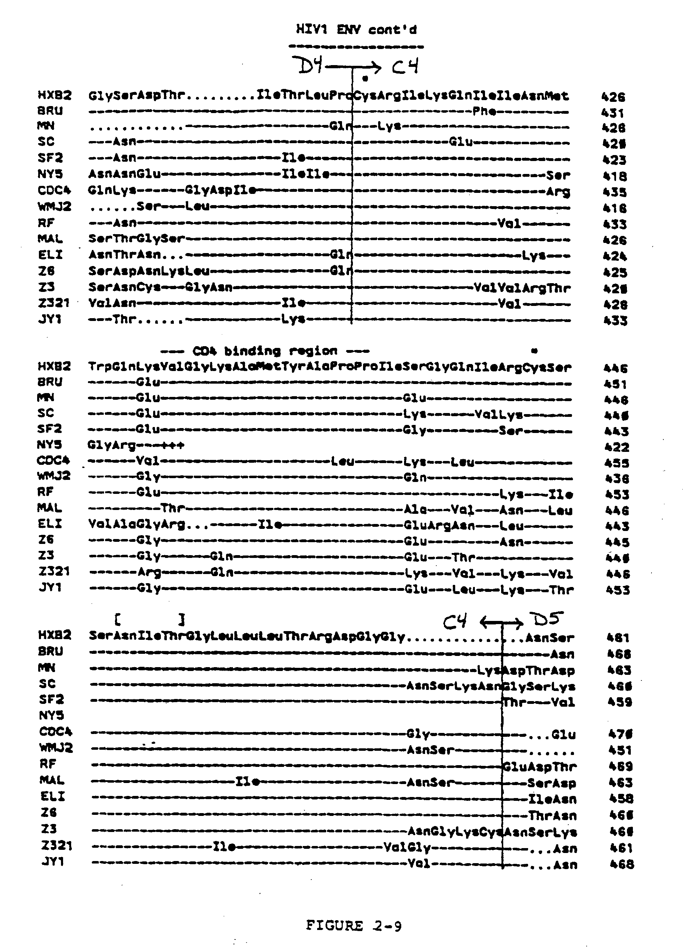

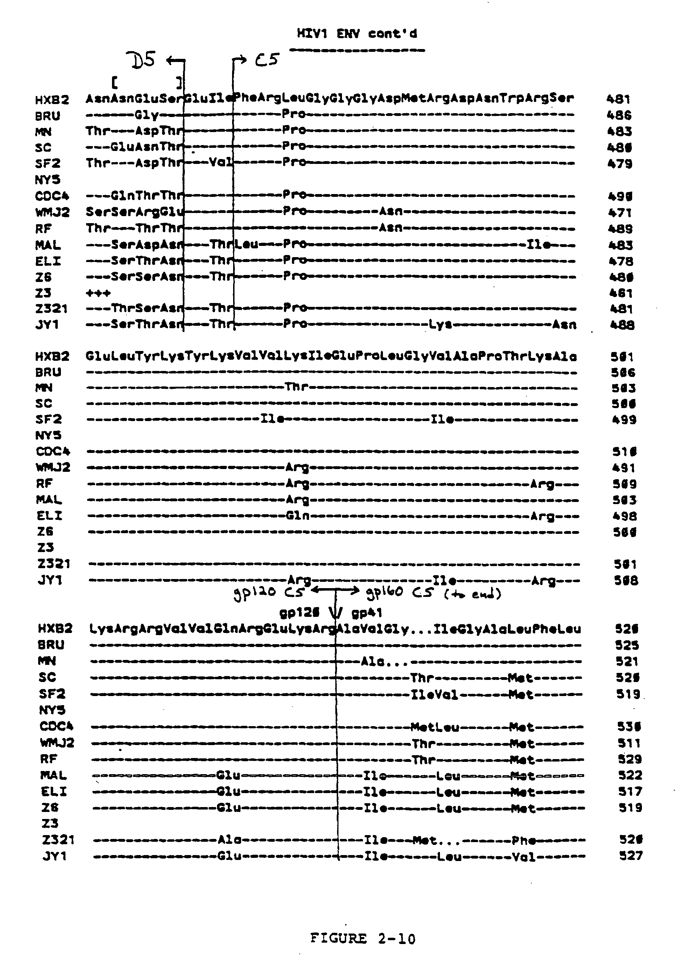

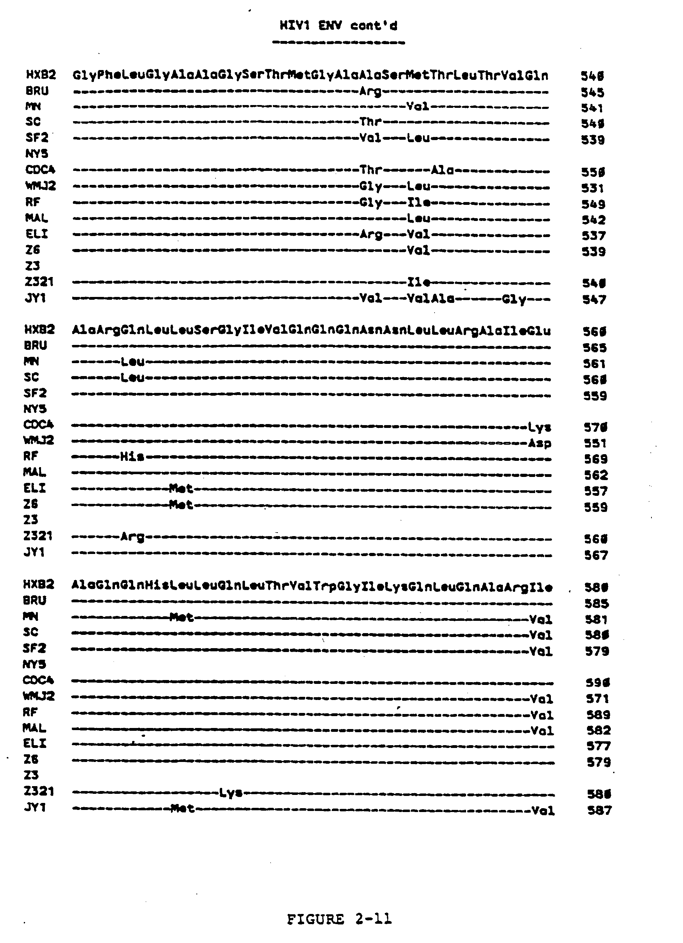

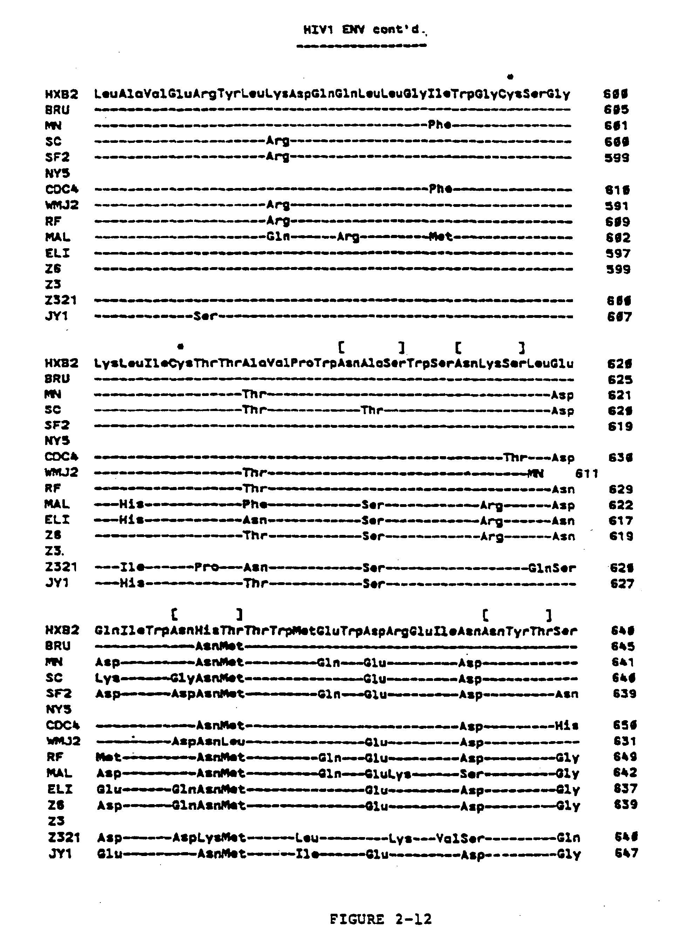

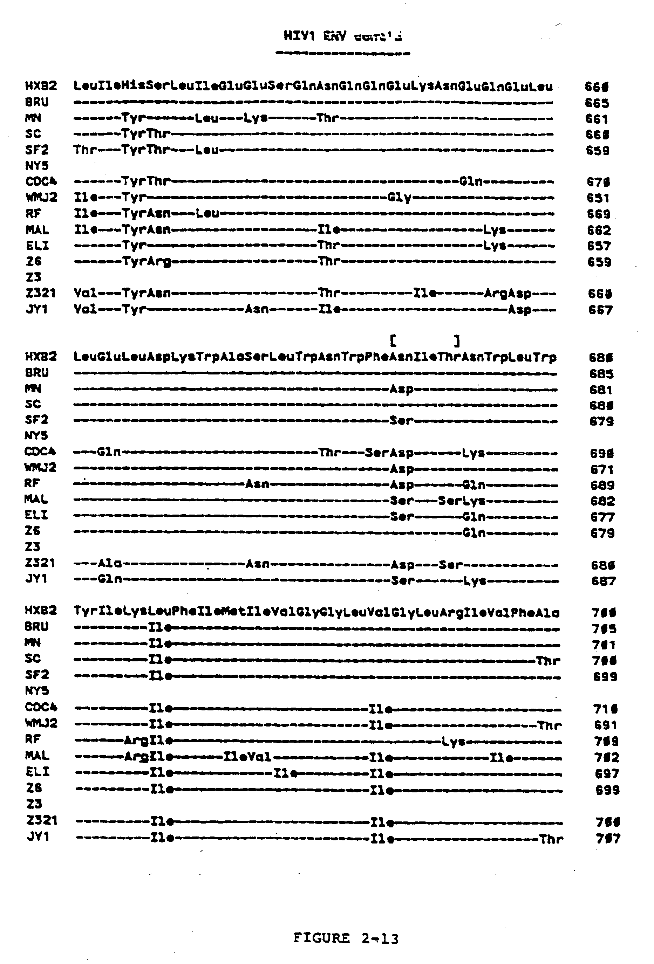

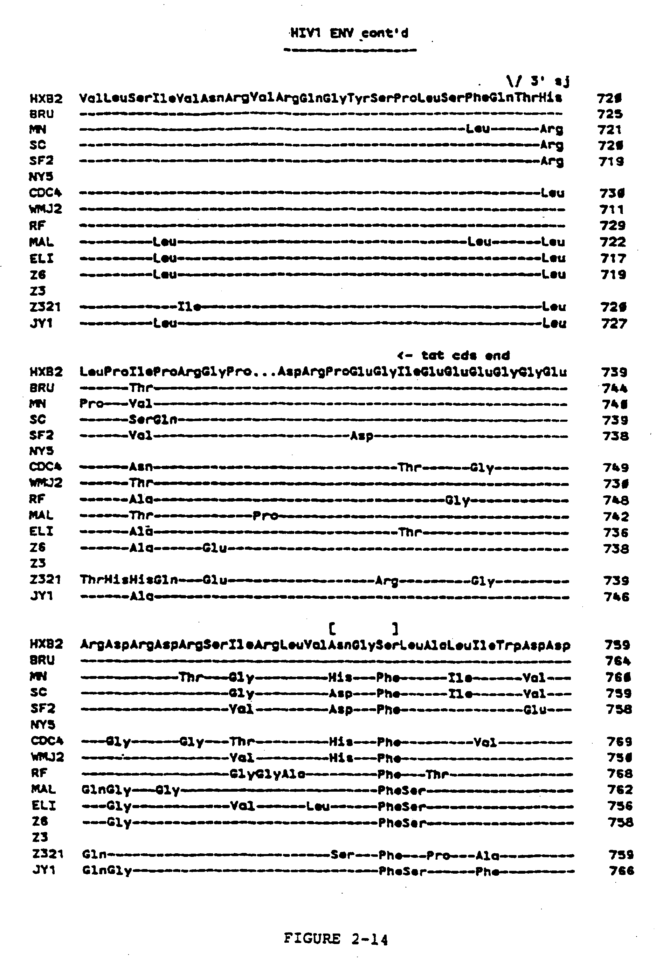

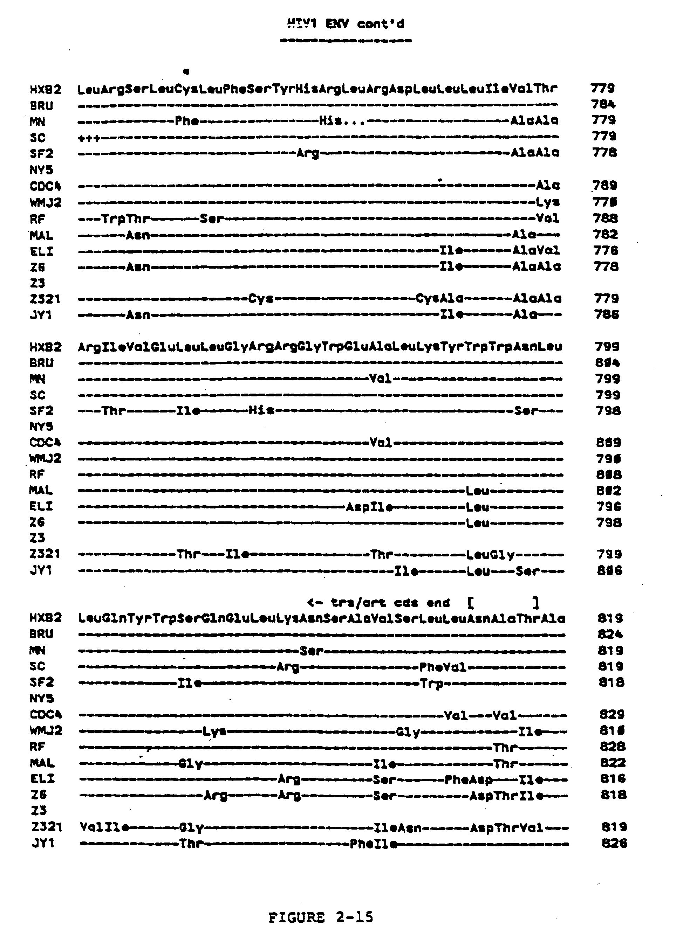

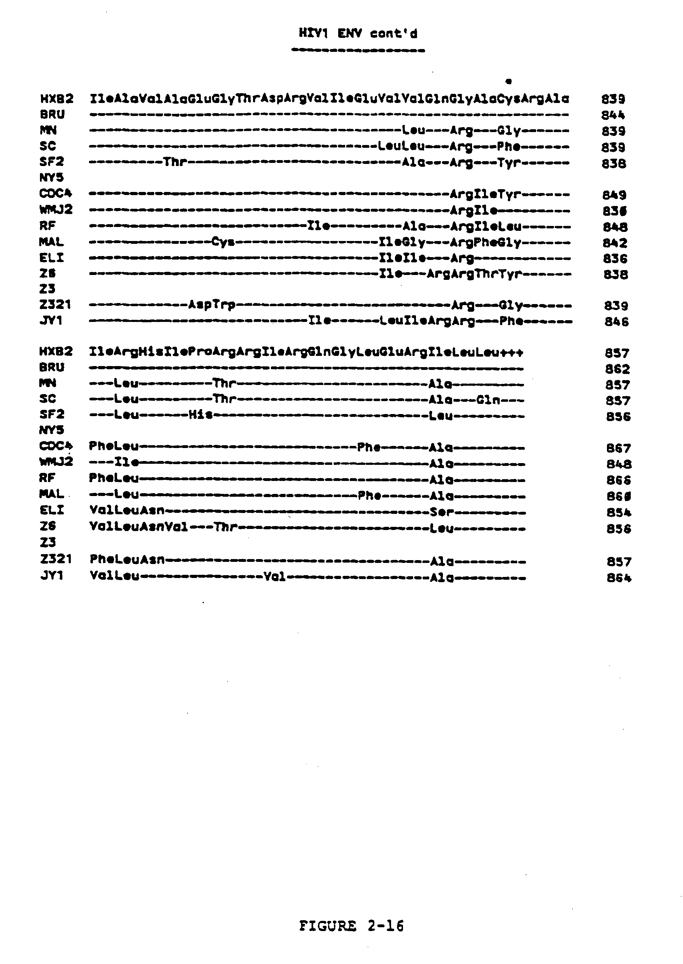

Figure 2 is a table of aligned amino acid sequences for various HIV-1 isolates with the constant (C) and variable (D) domains indicated. Potential N-linked glycosylation sites for the HXB2 sequence only are indicated by [ ]; cysteine residues have * above them in this figure. This sequence data was published in Human Retroviruses and AIDS 1988, A Compilation and Analysis of Nucleic Acid and Amino Acid Sequences, edited by Gerald Myers et al., published by the Theoretical Biology and Biophysics Group, T-10, Mail Stop K710, Los Alamos National Laboratories, Los Alamos, New Mexico, 87545. There is also a 1989 version edited and published by the same source.

Figure 3A is a graph showing product fractions as obtained in a purification step using a phenyl HIC column.

Figure 3B is a graph showing product fractions as obtained is a purification step using an ether HIC column.

Figure 3C is a graph showing product fractions as obtained in a purification step using gel filtration chromatography.

Figure 4 is a graph showing formation of a CD4-gp120 complex using gel filtration HPLC.

Figure 5 is a graph showing HIV-ZR6 neutralization data from baboon 2964 sera analyzed after 0, 5, 6, 7, 8, and 9 immunizations with gp120.

Figure 6 is a set of graphs showing neutralization titers of all serum samples from Example 6 immunized baboon 2958 and gp120-immunized baboon 2964.

Figure 7 is a schematic diagram of primate interrupted immunization regimen design.

### DESCRIPTION OF THE SPECIFIC EMBODIMENTS

### Example 1

### Mutagenesis and expression of HIV gp120 in mammalian cells.

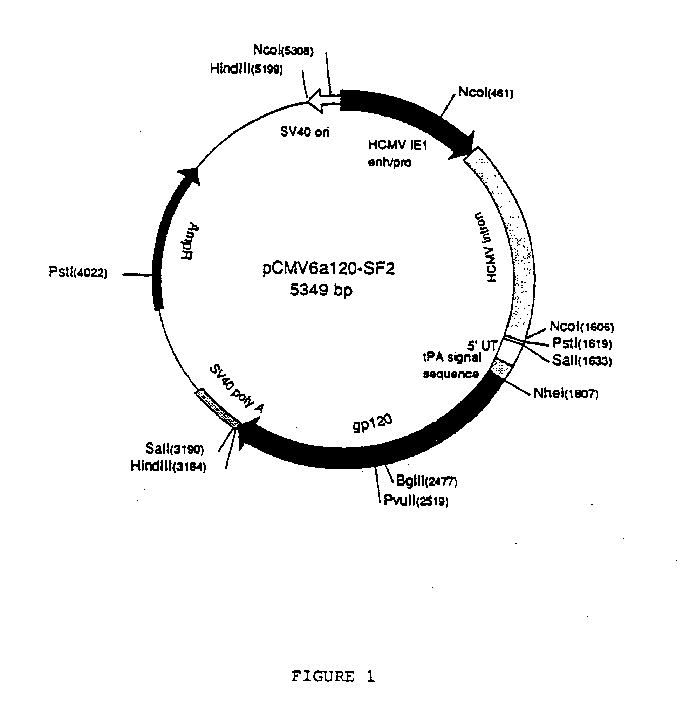

The envelope gene encoding gp160 of HIV-SF2 was engineered for expression of gp120 sequences by the introduction of a stop codon following Arg509 at the gp120 natural -gp41 processing site. The 5' end of the gene was modified to insert an Nhel restriction endonuclease site 5' to the sequences encoding Glu31, so that the natural signal sequence could be replaced by other signal sequences to test for improved secretion from mammalian cells. In order to produce gp120 as a secreted glycoprotein in mammalian cells, the HIV signal sequence and 5' untranslated sequences were replaced with those from human t-PA, mutagenized to place an Nhel site near the 3' end of the tPA signal DNA to encode Ala Ser. The resulting gene construct was fused to a series of promoters. Transient expression of gp120 was evaluated following transfection of the expression vectors in COS-7 cells and comparisons of levels of secreted gp120 by goat-capture ELISA (described below) and western blot. Highest levels of expression were seen using the CMV IE-1 promoter, at least 50-fold higher than with the SV40 early promoter. For construction of permanent cell lines the expression plasmid pCMV6aSF2-120 (Figure 1) was cotransfected with a dhfr expression plasmid using calcium phosphate coprecipitation into CHO dhfr-cells (dg44; see below). The resulting cell lines were characterized by screening clones with the gp120 goat-capture ELISA. Highest expressing cell lines were amplified in methotrexate in pools. Clones were isolated at the 0.1 mM level. Using purified protein as a standard, cell lines were shown to be secreting gp120 in the 5 mg per liter range at the T flask level.

The cells used for expression of the gp120 gene were originally obtained by Dr. Leslie Rall of Chiron Corporation, in September, 1985, at approximately 100 passages. These cells were originally isolated by Dr. Gail Urlaub and Dr. Lawrence Chasin at Columbia University, New York and are described in Urlaub et al., Cell (1983) 33: 45. The cells were designated as DG44. They are derived from Chinese hamster ovary (CHO) K-1 cells that were made dihydrofolate reductase deficient (dhfr⁻) by virtue of a double deletion.

The CHO dhfr⁻ cells were cultured continuously in the following medium: Hams F-12 medium supplemented with 10% dialyzed fetal calf serum, 200 mg/mL of streptomycin. The medium and serum were obtained from the University of California, San Francisco Cell Culture Facility, San Francisco, CA. All other ingredients were supplied by Sigma Chemical Co., St. Louis, MO. Cells were maintained by passaging two times a week with a 1:10 split in T-75 flasks.

For storage, aliquots of cells were frozen in fetal calf serum (FCS), 10% dimethyl sulfoxide (DMSO) and stored at -80RC in the gas phase of liquid nitrogen. For this purpose, T-75 flasks of cells were grown to confluency (approximately 10⁷ cells per T-75 flask). Cells were trypsinized, centrifuged and resuspended in ice-cold 10% DMSO in FCS at a concentration of about 5 x 10⁶ cells/mL. One mL aliquots were transferred to cryopreservative vials. When cells were required, an aliquot was thawed in a 37RC water bath and cells were seeded in T-75 flasks for continuous culturing and passage.

The two assays used as described above for detecting HIV-1 envelope-related antigens were carried out in the following manner. For both assays, purified CHO-derived gp120 was used as a standard, using two-fold dilutions from 200 ng/ml to 0.195 ng/ml.
(a) goat capture ELISA: The capture reagent for this assay was protein-A-Sepharose-affinity-purified immunoglobulin from a goat that had been hyperimmunized with purified env-2-3 (SF2), which is described below, a non-glycosylated polypeptide produced in yeast corresponding the amino acid sequence of gp120 of the HIV-SF2 virus isolate. The reagent used to detect captured antigen was a polyclonal antiserum raised in rabbits to the same antigen. Plates were coated with 5 mg/mL of goat immunoglobulin to env-2-3 (SF2), incubated with dilutions of viral lysate or mammalian-derived gp120 antigens and then the captured antigen detected by the rabbit polyclonal antiserum to env-2-3 (SF2) diluted 1/100 followed by conjugate and ABTS substrate.
(b) Human capture ELISA: This assay is identical to the "goat capture ELISA" described in a above except that the capture reagent was protein-A-Sepharose-purified immunoglobulin from human sera obtained from HIV-1 seropositive blood donors.

### Example 2

### Cellular Production.

One cell line, CHO-A-6a120-145-0.1-22, obtained as described in Example 1, was chosen for production in roller bottles in media with reduced serum and no methotrexate. Roller bottle cultures (850 cm²) were established and expanded to confluency in medium (Delbecco's Modified Eagle's Medium and Ham's F-12, 1:1) supplemented with 6% fetal calf serum (FCS). For production, supplementation was switched to 1% FCS with 0.03% HB-CHO (Hana Biologics, Alameda, CA.). Conditioned medium (200 ml) was collected every 24-48 hours, stored at 2-8 C, pooled and clarified by filtration through 0.45 micron capsule filters (Gelman). Cells were maintained for more than two months in each of two production runs with no apparent loss of production of gp120. Expression levels ranged from 5 to 20 mg/L.

### Example 3

### Purification

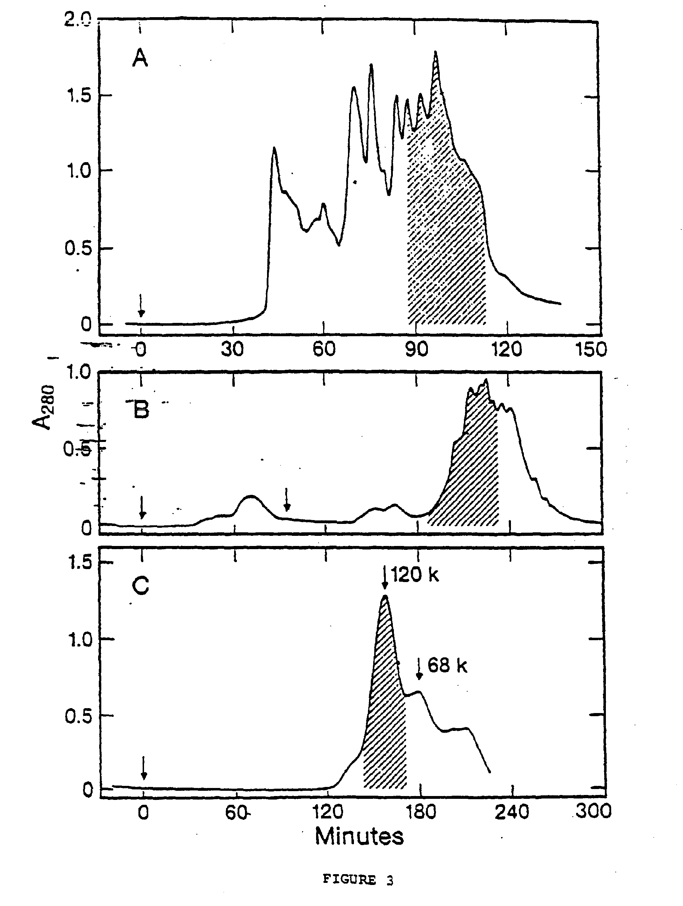

(1) Concentration. Concentration of the cell culture supernatant from Example 2 (40 L) was carried out using dead-end filtration (0.45 micron capsule filter, Gelman) and cross-flow ultrafiltration using a 30 K cutoff hollow fiber ultrafilter (AG Technology #UFP-30-C-6; 6 ft.² and 0.5 mm fiber i.d.) driven by a positive displacement pump (Waukesha #18). Permeation rate was approximately 150 ml/min at a recirculation rate of approximately 12 L/min and a pressure of 26 psi. Filtration continued until the retentate volume reached 1-2 L. The filtration steps were carried out in a cold room at 2-8°C. The ultrafiltration concentrate was a brown, clear liquid.
(2) DEAE Chromatography. The concentrate was applied to an ion exchange column (11.4 cm diam x 15 cm) packed with DEAE Sephadex A-50 (Pharmacia) was equilibrated in Buffer (0.02 M Tris-CI, pH 8.0, 0.1 M NaCI) at a flow rate of 35 ml/min at room temperature. The ultrafiltration concentrate was brought to a volume of 2 L and a conductivity of 1.4 mS by addition of sodium chloride (4 M stock solution). The unadsorbed fraction containing the product was collected in 250 ml fractions using an Isco Foxy® fraction collector. Serum albumin, other proteins and the bulk of the brown colored material bound to the column and were eluted with a step gradients of 1 M NaCI. These fractions contained a small but variable amount of product; no attempt was made to recover product from the bound fraction. The DEAE Sephadex A-50 resin was discarded after each use. The pass-through fraction has been shown to contain the bulk of the product by ELISA assay. At this stage of purity it was difficult to locate the diffuse gp120 band on an SDS gel.
(3) Phenyl Hydrophobic Interaction Chromatography. The DEAE fraction was brought to 40% saturation in ammonium sulfate by addition of solid ammonium sulfate. After thorough mixing a small amount of precipitate was removed by centrifugation. A TSK Phenyl-5PW HIC column (5.5 cm diam x 20 cm) was washed with at least two volumes of water using a Gilson preparative HPLC. Then the column was equilibrated with two or more volumes of Buffer A (0.02 M sodium acette, pH 5.0, 40%-saturated ammonium sulfate). Column equilibration was verified by conductivity measurement of the effluent. The supernatant fraction after addition of ammonium sulfate was applied to the column by pumping through Pump A at 30 ml/min, then the column was washed with Buffer A until the baseline stabilized (usually about 15-20 min). A gradient was run to 0.02 M sodium acetate, pH 5.0, over 40 min to elute the product. Fractions under the OD peak were assayed by SDS gel electrophoresis using a Pharmacia Phast® system to locate the product. At this stage of purity the gp120 band was clearly discernible. Product-containing fractions were pooled for the next stage of chromatography (see Fig. 3A).
   (d) Ether Hydrophobic Interaction Chromatography. A second HIC step was carried out on a TSK Ether-5PW HPLC column (5.5 cm diam x 20 cm) following the same procedure used for the phenyl HIC column. The column was washed with at least two column volumes of water, then equilibrated in Buffer A (40%-saturated ammonium sulfate, 0.02 M sodium acetate, pH 5.0). The product pool from the phenyl column was brought to a conductivity of 165 S/cm by addition of ammonium sulfate, followed by centrifugation for 10 min at 12,000 rpm. The sample was loaded and eluted as described above using a 40 min gradient from 100% Buffer A to 100% Buffer B. Product-containing fractions (see Fig. 3B) were located by SDS gel electrophoresis on a Phast® system, then pooled for gel filtration chromatography. The gp120 peak from the ether column was mainly gp120 with smaller amounts of lower molecular weight contaminants. These contaminants were resolved by gel filtration chromatography (below).
(5) Gel Filtration Chromatography. The ether HIC fraction was concentrated on an ultrafiltration membrane (Amicon YM-30) to a protein concentration of approximately 10 mg/ml as measured by A-280 assuming an extinction coefficient of 0.6 = 1 mg/ml, then diafiltered against at least five volumes of 0.1 M sodium phosphate, pH 6.9. Sample was applied to a gel filtration column (Superdex®200, Pharmacia, 1.6 cm diam x 60 cm) at a total protein concentration of not more than 10 mg/ml in a volume of not more than 4% of the column volume and eluted with 0.1 M sodium phosphate, pH 6.9. Fractions of 1 ml were collected, subjected to SDS gel electrophoresis with Coomassie Brilliant Blue R350 staining on a Phast system and to get filtration HPLC on a DuPont GF-450 column (running buffer: 0.2 M sodium phosphate, pH 6.7, 1 ml/min) to locate dimer-containing fractions, then pooled. The leading edge of the gp120 peak contained pure gp120 while the trailing edge was rechromatographed on the gel filtration column. The product pool was concentrated on an Amicon YM-30 membrane, diafiltered against 5 volumes of distilled water, and lyophilized for at least two days at a pressure of less than 10 microns.
(6) Summary of Purification Results. Table 1 summarizes the results of a typical purification starting with 40 liters of cell culture supernatant. These data show that a 250-fold purification is achieved with a yield of 20-25%. The product appeared as a broad band migrating at H120 KD in an SDS gel. Densitometry revealed 80-90% of the staining intensity was under the gp120 band. This probably represents a minimum estimate of the purity of this preparation because gp120 binds stain poorly. Approximately 7-fold less Coomassie Brilliant Blue was bound per microgram protein compared to BSA. The appearance of the gel band was not altered by pretreatment of the sample with 2-mercapotethanol or dithiothreitol, showing that the protein is not internally cleaved. Reverse phase HPLC analysis at elevated temperature also suggested that the purity of the product exceeded 90%.

**Table 1**

| SF2 rgp120 Purification Table | | | | |
|---|---|---|---|---|
| Step | Volume | Protein | rap120 | Purity |
| 1. Culture Supernatant | 40.0L | 55.g | 210.mg | 0.4% |
| 2. UF Concentrate | 3.76 | 44.9 | 180 | 0.4 |
| 3. DEAE | 4.75 | 8.55 | 140 | 1.6 |
| 4. Phenyl HIC | 0.724 | 0.996 | 150 | 15 |
| 5. Ether HIC | 0.260 | 0.354 | 110 | 31 |
| 6. Gel Filtration | 0.020 | 0.053 | 48 | 90 |

### Example 4

(1) Comparison of Purified SF-2 rgp120 with Viral gp120. Purified gp120 was subjected to SDS polyacrylamide gel electrophoresis to assess size and purity. The protein migrated at the predicted location for a 120 K protein with a broadly staining band characteristic of glycoproteins. This broad band is consistent with expected carbohydrate heterogeneity at the 22 predicted N-linked glycosylation sites, which has been described for other isolates. To compare the recombinant gp120 with that found in virions, lysates of HIV-SF2-infected HUT-78 cells were prepared and examined by western blot with HIV-positive human and gp120specific animal sera. Patterns observed were consistent with conserved conformation.
(2) N-Terminal Sequencing. The amino-terminal amino acid sequence was determined by automated Edman degradation. The observed and expected sequences were:

| | |
|---|---|
| observed | E K L W V T V Y Y G V P V W K... |
| expected | T E K L W V T V Y Y G V P V W K... |

This sequence confirms that the heterologous signal was correctly processed by the signal peptidase, following serine of the signal, and that the protein is not fused to any additional amino acids. This sequence lacks the N-terminal threonine found on viral gp120 from the HTLVIIIB isolate (Robey et al., PNAS (1986) 83: 7023-7027). The N-terminal amino acid sequence matches the HIV-SF2 envelope sequence predicted from the DNA sequence of this isolate for at least the first fifteen amino acids.
(3) Amino Acid Composition. Amino acid analyses were performed on five lots of gp120 purified as described in Example 3. The average of these values agreed with the composition expected from the DNA sequence within experimental error for all amino acids except ile (33.5 observed vs. 39 expected) and ser (32.7 observed vs 24 expected). The ser value was variable within the five lots and probably represents a serine-rich contaminant.
(4) Native Gel Electrophoresis and IEF. Charge heterogeneity of gp120 was evident in isoelectric focusing experiments and in native gel electrophoresis. Isoelectric focusing revealed the presence of multiple bands within the envelope pH 5 to 7. The protein migrated as a single broad band in a nondenaturing polyacrylamide gel.
(5) Gel Filtration HPLC. The molecular weight of recombinant gp120 was 120 K in the presence of SDS; molecular weight in the absence of SDS was measured by gel filtration HPLC. At neutral pH in medium ionic strength buffers, purified gp120 eluted as a single major peak with a retention volume corresponding to a molecular weight of H130 K. A small amount of dimer was also present; the fraction of dimer increased to 10-20 of the total gp120 upon storage in solution. The dimer fraction was isolated at the gel filtration step and analyzed separately. This fraction migrated as a monomer when analyzed by SDS gel electrophoresis in the presence of reducing agent but as a dimer in the absence of reducing agents (2-mercaptoethanol or dithiothreitol) so it was probably linked by disulfide bonds. The amino acid composition of the dimer fraction was indistinguishable from that of the monomer fraction. The dimer fraction also bound CD4 when tested by the radioimmune precipitation assay. The gp120 gel filtration HPLC peak was broader than one would expect for a protein of this molecular weight. The extra peak width obtained for gp120 can be attributed to heterogeneity in the carbohydrate moiety. The high molecular weight of gp120 relative to the impurities present made it possible to use gel filtration HPLC as a purification assay after the phenyl HIC step. It was routinely used as an assay at the gel filtration step to eliminate from the product pool the fractions containing gp120 dimers.
(6) CD4 Binding. The CD4 used in this example was recombinant, soluble CD4 derived from a CHO cell line transfected with an expression plasmid encoding the full external domain. Details on CD4 production for use as a binding standard are set forth in Example 5. Binding experiments were done by radioimmune precipitation by gel filtration HPLC.
(a) General Techniques of Radioimmune Precipitations. Confluent monolayers of cells producing (for example) gp120 were labelled in Dulbecco's modified Eagle medium without cysteine and methionine (cys-met-DME). Five ml of cys-met-DME with 100 mCi/ml each ³⁵S met and cys, were added to each T75 flask for 6-8 hours. Labelled samples were harvested, centrifuged to remove cells, and stored at -80C until use. Samples to be precipitated were adjusted to 1X lysis buffer [0.1 M NaCI, 0.02 M tris pH 7.5, 1 mM EDTA, 0.5% NP40, 0.5% deoxycholate, 0.1% bovine serum albumin (BSA), 1 mM phenyl methyl sulfonyl fluoride (PMSF), 17 mg/ml aprotinin]. Samples were precleared with one tenth volume normal goat serum for 30 minutes at 4C, followed by 30 minutes precipitation with Protein A Sepharose (PAS) (1/2 volume 20% suspension) at 4RC. Immunoglobulin from hyperimmunized animals or HIV-positive human serum samples was affinity purified using PAS by standard techniques. Sera were titrated for the best signal to noise ratio; most immunoglobulin fractions were used at 5-10 mg per sample. Immune precipitations were 1-12 hours at 4C, depending upon the volume of the sample, followed by 1 hour with PAS. All samples were adjusted to the same volume within an experiment The PAS was washed with lysis buffer without BSA, followed by 0.12 M Tris pH7, and the pellets were solubilized in 1 Laemmli sample buffer, boiled, and applied to gels. Gels were treated with En³Hance®, dried, and fluorographed.
(b) CD4 Binding By Radioimmune Precipitation. CD4 was labelled with ³⁵S as described above, and the concentration of CD4 was determined using a capture ELISA employing a monoclonal antibody and polyclonal rabbit serum raised against CD4. For coprecipitation experiments, CD4 was added in increasing amounts to a fixed amount of gp120 (1 mg) to determine the saturating amount, and then coprecipitated with anti-gp120 antisera. This amount of CD4 was used for gp120 titration experiments. Labelled CD4 was precleared with normal serum, as described above. Following preclearing of the labelled component, CD4 and gp120 were complexed for 1 hour at 4C, then antibody against the unlabelled component was added (10 mg per sample) for 1 hour at 4RC. OKT4 was purchased from Ortho Diagnostics. PAS was added for 1 hour at 4RC, and the complexes were washed and prepared for electrophoresis as described above.
   Gp120 pre- and post-purification were both effective in binding to CD4 by this assay, as shown by equivalent band intensities for equivalent amounts of added gp120. A nonglycosylated analog of gp120 produced in yeast (env 2-3; see U.S. Patent Application No. 138,894, filed December 24, 1987) was unable to bind to CD4 in this assay. The dimeric form of gp120 isolated from the Superdex®200 column also bound CD4 by this assay. Saturation of binding was determined graphically. From the half-saturation levels a K_{d} of 6.9 nM was measured.
(c) CD4 binding by Gel Filtration HPLC. Purified gp120 and unlabeled CD4 were mixed in a volume of 60 ml containing 0.3 M potassium phosphate, pH6.8. After mixing, a portion of the sample (45 ml) was injected onto a DuPont GF-450 gel filtration HPLC column with a Waters WISP 712 sample injector run in 0.4 M potassium phosphate, pH 6.8 at 1 ml/min. The optical density was monitored at 215 nm and data was recorded using Waters Maxima 820® chromatography software.

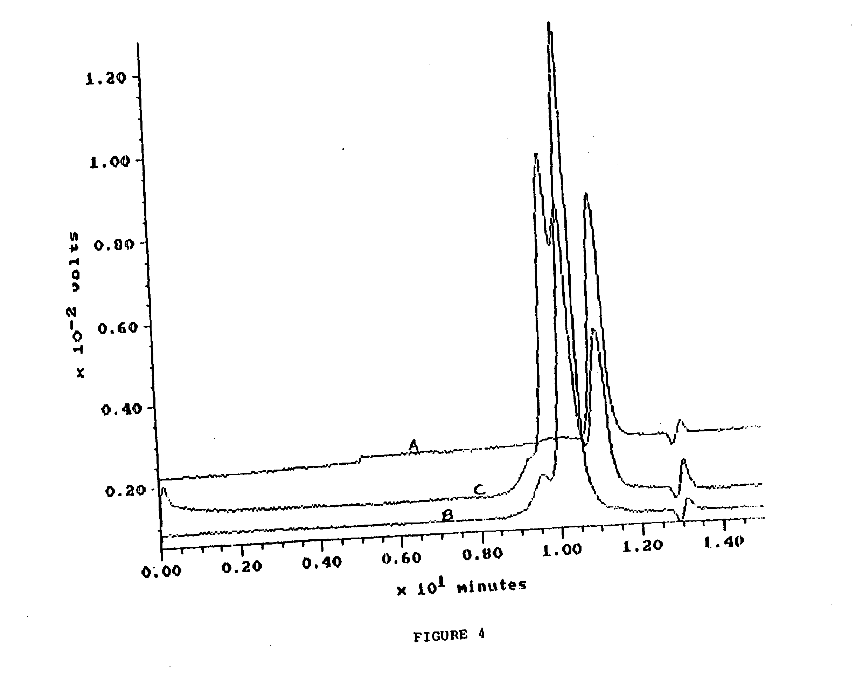

When CD4 and gp120 were applied separately to a gel filtration HPLC column each component gave a single peak at the expected elution time (See Fig. 4; Trace A is CD4 alone, Trace B is gp120 alone). When the components were mixed together before chromatography, a new peak appeared at an elution time corresponding to 160 K and the peaks at 120 K and 40 K diminished (Fig. 4; Trace C) This result provides direct physical evidence of the formation of a 1:1 complex between CD4 and gp120. Additional experiments were done with varying ratios of CD4 and gp120 and at different concentrations of the reactants. The results of these experiments supported the existence of a high affinity complex between one molecule of CD4 and one molecule of gp120.

### Example 5

CHO cells were cotransfected with AD-dhfr and an expression plasmid encoding soluble recombinant human CD4 (full external domain). The expression vector was constructed by cloning a CD-4-encoding sequence, a gift of Dr. D. Littman of UCSF, into the vector pCMV6a (pCMV6a120-SF2 minus the gp 120 coding sequence). A cell line secreting soluble CD4 was isolated. The resulting cell line, identified as CHO ST4.2 is available publicly as previously described. The cloned gene, ST 4.2, encodes 380 amino acids corresponding to the four extracellular domains to the transmembrane boundary. The purification process for this protein involves two columns. First, the CHO cell supernatant was loaded onto and eluted from an S. Sepharose cation exchange column. One liter CHO supernatant was diluted to 15 L with double distilled water and loaded onto 300 ml swollen resin equilibrated in 0.2X PBS/2.5 mM EDTA, pH 7.0 (conductively 3.6 ohm⁻¹cm⁻¹) at a load rate of 3.6 L/hr at room temperature. The column was rinsed with 500 mL 0.2X PBS/2.5 mM EDTA and 200 mL 50 mM NaCI 0.2X PBS 12.5 mM EDTA. Elution was with 1L 200 mM NaCI 10.2X PBS 12.5 mM EDTA. The eluate from this S. Sepharose column was then run over a monoclonal antibody affinity column. The monoclonal antibody (25-10-F5.5C1; hereafter referred to as 25-10-F5) used for this purification recognizes a conformational epitope in the amino-terminal half (within the first two immunoglobulin-like domains) of the extracellular region of CD4. Other antibodies with specificity for any epitope within the same domains should be equally effective. The S. Sepharose eluent was filtered (0.45 micron) and loaded onto the affinity column at 1 ml/min or less. The loaded column was rinsed with distilled water (25 X resin volume) and eluted with 5 mM triethylamine formate, 10 ml elution buffer per 4 ml of gel resin. The pH of the mAb eluent was adjusted to pH 7 with 1 M Tris (pH 8.0). The fractions eluted from the affinity column were the dialyzed and concentrated. Table 1 shows the yield at each step of the purification procedure.

**TABLE 1**

| Purification table for ST4.2 CD4 produced in genetically engineered CHO cells | | | | | | | | |
|---|---|---|---|---|---|---|---|---|
| Fraction | Volume (ml) | Concentration (mg/liter) | Protein (mg) | mg CD4/ liter | mg CD4/ mg protein | Total CD4 (mg) | Yield (%) | Fold purification |
| Supernatant | 486 | 1020 | 496 | 30.8 | 0.03 | 14.7 | -- | 1 |
| S-sepharose eluate | 1000 | 11.9 | 11.9 | 11.12 | 0.93 | 11.12 | 76 | 31 |
| Affinity column eluate | 30 | 0.183 | 5.49 | 274 | 1.00 | 8.22 | 56 | 34 |
| Affinity column flow through | 1000 | N.D.^{a} | N.D. | 0.05 | N.D. | 0.05 | 0.4 | -- |

| | | | | | | | | |
|---|---|---|---|---|---|---|---|---|
| a. N.D. = Not determined. | | | | | | | | |

The levels of active CD4 in the various fractions were determined by using a capture ELISA employing the monoclonal antibody 25-10-F5 as the capture reagent and a rabbit polyclonal antisera raised against purified ST4.2 as the detecting reagent. The fractions and flow through from each column were compared to the initial supernatant and a known CD4 standard. This allowed quantitation of how much active CD4 was recovered at each step. It also allowed one to estimate the increase in purity following each step of the purification. This was done by comparing the total amount (milligrams) of active CD4, as determined by the ELISA, with total milligrams of protein, as determined by a Pierce protein microassay. Using these techniques the yield for the S. Sepharose column was shown to be 76% and the affinity column to be 74%, giving an overall yield of 56%. Note that the S. Sepharose column alone resulted in a 31-fold purification, yielding a solution that was 93% CD4 after just the first step. The affinity column increased the purity of the S. Sepharose eluent to essentially homogeneity.

The purity of these final fractions was analyzed in two ways. First, the protein was run on a 12% SDS gel and stained with Coomassie brilliant blue. This visual analysis indicated that the protein was highly purified; at least 95% of the final product was CD4. An amino acid analysis was performed on ST4.2 samples purified according to this protocol also indicated that the material was highly purified.

The gp120 binding ability of purified ST4.2 was analyzed both by ELISA and using a gp120 column. ST4.2 could be coated onto microtiter plates and would retain gp120 binding activity. To test gp120 binding of the various lots of CD4, microtiter plates were incubated with various concentrations of CD4 from each lot and then added a single concentration of gp120 to all wells. Bound gp120 was detected with a rabbit polyclonal antiserum to gp120 (Rb anti-env2-3 serum). A strong signal was seen which titered out as the amount of ST4.2 coated onto the plate decreased. Gp120 binding was also assessed for two of the lots by running the purified ST4.2 over an affinity column of gp120. An initial solution of 10 mg/ml ST4.2 was loaded on to the column. The CD4 content of each fraction was determined by immunoblot analysis of the various fractions utilizing the polyclonal rabbit antiserum to ST4.2 discussed above. These results indicated that close to 100% of the CD4 immunoreactive material was absorbed to the gp120 on the column matrix and eluted as a specific peak.

Native and denatured ST4.2 were coated onto microtiter plates and the ability of various CD4-specific immunological reagents to recognize the two forms of the protein were compared. A rabbit polyclonal serum, prepared by immunization with purified ST4.2, recognized both native and denatured forms of CD4; OKT4A, which is known to recognize a conformational epitope, clearly reacted with native CD4, but did not react with the protein that had been denatured. The monoclonal antibody 25-10-F5 showed a pattern of reactivity similar to OKT4A.

Preparations of purified ST4.2 were stored at -80°C and 4°C and tested periodically for (1) immunoreactivity with the rabbit polyclonal antiserum that recognizes both native and denatured CD4, (2) recognition by OKT4A and 25-10-F5, which only react with native CD4 and (3) gp120 binding. A significant loss in activity assessed by OKT4A and 25-10-F5 monoclonal antibody as well as gp120 binding was observed upon storage at 4°C. However, the material stored at -80°C retained full activity. In addition, it has also been noted that purified ST4.2 looses activity upon repeated freezing and thawing.

### Example 6

An immunization experiment was carried out to compare production of neutralizing antibodies using a gp120 prepared as described above with retained conformation to other gp120 molecules whose conformation is known to be modified. A gp120 analog (env 2-3) prepared in yeast, which is denatured and non-glycosylated, was used as a comparison antigen. Both gp120 materials were derived from the same gene source, HIV-1 SF-2 isolate. Antibody production was measured in baboons using the immunization schedule shown in Table 2.

**TABLE 2**

| Group # | Animal Number(s) | Adjuvant | | Antigen | | Volume Per Site | Sites Per Animal | Injection Route |
|---|---|---|---|---|---|---|---|---|
| | | Name | Dose | Name | Dose | | | |
| | | | | | | | | |
| 1 | (3) | MTP-PE | 250mg | gp120/SF2 | 55mg | 0.5mL | one | IM/thigh |
| | 2951,2953,2964 | in ICFA (SY) | | | | | | |
| | | | | | | | | |
| 2 | (3) | MTP-PE | 250mg | env2-3/SF2 | 25mg | 0.5mL | one | IM/thigh |
| | 2952,2957,2958 | in ICFA (SY) | | | | | | |
| | | | | | | | | |
| 3 | (3) | MTP-PE | 250mg | gp120/SF2 | 55mg | 0.5ml | one | IM/thigh |
| | 2949,2954,2966 | in S (MF/KE) | | | | | | |
| | | | | | | | | |
| 4 | (3) | MTP-PE | 250mg | env2-3/SF2 | 25mg | 0.5ml | one | IM/thigh |
| | 2950,2956,2967 | in S (MF/KE) | | | | | | |
| | | | | | | | | |
| 5 | (2) | Alum | 0.8mg | gp120/SF2 | 55mg | 0.5ml | one | IM/thigh |
| | 2955, 2965 | | | | | | | |
| | | | | | | | | |
| 6 | (2) | Alum | 0.8mg | env2-3/SF2 | 25mg | 0.5ml | one | IM/thigh |
| | 2947, 2948 | | | | | | | |

Immunogens were prepared in the following manner:
(1) Groups 1 and 2: Add one part antigen (gp120 or env 2-3) to two parts incomplete Freund's Adjuvant (ICFA), mix by syringe, and inject 500 ml per animal.
(2) Groups 3 and 4: Warm vial of antigen/MTP-PE adjuvant to roomtemperature, vortex for one minute, and inject 500 ml per animal within 30 minutes (re-mix as needed).
(3) Groups 5 and 6: Warm vial of antigen/alum to room-temperature, vortex for one minute, and inject 500 ml per animal within 30 minutes (re-mix as needed).

Immunization was carried out at the beginning of the experiment and at the 4th, 8th 12th and 20th week after start of the experiment. Blood samples were taken at the start of the experiment (pre-bleed) and at the times indicated in the tables (below) which report results.

The results are summarized in the attached Tables 3 and 4. The env 2-3-immunized animals show neutralizing activity against the homologous isolate, HIV-SF-2, in all adjuvant groups, and in one adjuvant group (IFA-MTP) neutralization against HIV-MN (3 of 7 animals total). Thee is one animal that shows detectable neutralization against HIV-HTLV-IIIB with this antigen.

In contrast, all of the gp120-immunized animals show neutralizing activity in all three adjuvant groups against HIV-SF2 and HIV-MN, both after four and after five immunizations. Six of 8 gp120-immunized animals also have significant neutralizing activity against HIV-HTLBIIIB, and the animals are from all three adjuvant groups.

**TABLE 3**

| Neutralization Titers of Baboons Immunized with Env 2-3 (SF2) | | | | | | | | |
|---|---|---|---|---|---|---|---|---|
| Animal | Adjuvant | | Neutralization titers^{a} | | | | | |
| | | Virus Serum | SF2 | | MN | | HTLVIIIB | |
| | | | Bleed7^{c} | Bleed 12^{d} | Bleed 5^{b} | Bleed 12^{d} | Bleed 5^{b} | Bleed 12^{d} |
| 2952 | 1^{f} | | 10 | 7.5 | 4 | 5 | 0 | 0 |
| 2957 | 1 | | 20 | 20 | 0 | 1.8 | 0 | 0 |
| 2958 | 1 | | 20 | 77 | 200 | 170 | 0 | 4.9 |
| | | | | | | | | |
| 2950 | 2 | | 6 | 0 | 0 | 0 | 0 | 0 |
| 2956 | 2 | | 6.5 | 0 | 0 | 0 | 0 | 0 |
| 2967 | 2 | | 12 | 4.5 | 0 | nt^{e} | 0 | nt^{e} |
| | | | | | | | | |
| 2947 | 3 | | 11 | nt^{e} | 0 | 0 | 0 | 0 |
| 2948 | 3 | | <4 | nt^{e} | 0 | 0 | 0 | 0 |
| #positive/#animals | | | 8/8 | 4/6 | 2/8 | 3/7 | 0/8 | 1/7 |
| | | | | | | | | |
| Mean | | | 11 | 18 | 26 | 25 | 0 | 0.7 |

| | | | | | | | | |
|---|---|---|---|---|---|---|---|---|
| a. Neutralization assays were performed as follows: Virus stocks were titered for syncytial-forming units (SFU) on CEM-SS cells. Sera to be tested were heat-inactivated, diluted serially two-fold and mixed with 200 SFU for 1 hour at 37°C, then pipetted onto CEM-SS cells attached to microwells with poly-L-lysine for 1 hour at 37°C. Virus-serum mixtures were then removed and the cells fed with medium. Syncytia formation was scored at 5 days post infection. Neutralization was scored by calculating Vn(# of syncytia formed in test wells) divided by Vo(# of syncytia in virus alone wells) for each dilution of test sera, and Vn/Vo was plotted as a function of serum dilution. Titers reported are the inverse of the dilution that gave Vn/Vo<0.1 (>90% neutralization). b. Bleed 5 is at 10 weeks, two weeks following the third immunization. c. Bleed 7 is at 14 weeks, two weeks following the fourth immunization. d. Bleed 12 is at 23 weeks, three weeks following the fifth immunization. e. nt, not tested. f. Adjuvant 1 = IFA (incomplete Freund's Adjuvant) + 250mg MTP-PE Adjuvant 2 = MF101 (250mg MTP-PE) Adjuvant 3 = Alum | | | | | | | | |

**TABLE 4**

| Neutralization Titers of Baboons Immunized with gp120 (SF2) | | | | | | | | |
|---|---|---|---|---|---|---|---|---|
| Animal | Adjuvant | | Neutralization titers^{a} | | | | | |
| | | Virus Serum | SF | | MN | | HTLVIIIB | |
| | | | Bleed 7^{c} | Bleed 12^{d} | Bleed 5^{b} | Bleed 12^{d} | Bleed 5^{b} | Bleed 12^{d} |
| 2951 | 1^{e} | | 40 | 160 | 140 | 128 | >2 | 55 |
| 2953 | 1 | | 64 | 8 | 8 | 17 | 0 | 15 |
| 2964 | 1 | | >64 | 230 | 80 | >256 | 7 | 82 |
| | | | | | | | | |
| 2949 | 2 | | 16 | 9.5 | 4.5 | 9.5 | 0 | 0 |
| 2954 | 2 | | 16 | 13 | 8 | 24 | 0 | 0 |
| 2966 | 2 | | 65 | 27 | 32 | 29 | 0 | 4 |
| | | | | | | | | |
| 2955 | 3 | | 16 | nt^{e} | 5 | 40 | 0 | 15 |
| 2965 | 3 | | 7 | nt^{e} | 4 | 17 | 4.8 | 14 |
| #positive/#animals | | | 8/8 | 6/6 | 8/8 | 8/8 | 3/8 | 6/8 |
| | | | | | | | | |
| Mean | | | 36 | 75 | 35 | 65 | 1.7 | 23 |

| | | | | | | | | |
|---|---|---|---|---|---|---|---|---|
| a. Neutralization assays were performed as described in Table 1. b. Bleed 5 is at 10 weeks, two weeks following the third immunization. c. Bleed 7 is at 14 weeks, two weeks following the fourth immunization. d. Bleed 12 is at 23 weeks, three weeks following the fifth immunization. e. Adjuvants were as described in Table 1. | | | | | | | | |

Young adult (male/female) baboons (Papio) were immunized with 55 mg gp120 formulated in one of two adjuvants: aluminum hydroxide (alum, 0.8 mg per dose); or Incomplete Freund's Adjuvant plus 250 mg muramyl tripeptide (IFA-MTP). Animals were immunized approximately every four weeks and the sera were monitored for the loss of envelope-specific titer. Data summarizing the antigen-specific response for each animal in the study is set forth in Tables 5 and 6. Envelope specific titers peaked following each boost and then declined. Note that the alum and IFA-MTP titers differ by approximately tenfold. Baseline titers were reached after six months of rest, and the animals were then reboosted at monthly intervals. To measure the effectiveness of the envelope antibodies in virus neutralization, sera were tested in in vitro neutralization assays against both homologous HIV-SF2 and heterologous virus isolates. Sera were tested at points of known high envelope titers for virus neutralization, at weeks 10 (after 3 immunizations), 23 (after 5 immunizations) before the rest, and at week 57 (after 6 immunizations) after the boost following the rest. Two virus neutralization assays were employed, a p24gag inhibition assay described in Haigwood et al., AIDS Res. Hum. Retrov. (1990) 6;855-869 and Steimer et al, Vaccine (1988), H. Gimsberg et al., Editor, Cold Spring Harbor Laboratory Press, p 347-355, and an infectious center inhibition assay by Nara et al., AIDS Res. Hum. Retrov. (1987), 3:283-302. As illustrated in Table 5, neutralizing antibodies effective against HIV-SF2 and HIV-MN were generated after only three immunizations in both adjuvant groups, and titers were maintained or increased with further boosting. In Table 6, HIV-BRU- and HIV-HTLVIIIB-specific neutralizing antibodies were reproducibly observed after five and six immunizations; no titers versus HIV-ZR6 were observed after six immunizations. Overall, both the homologous SF2 and the heterologous neutralizing titers were higher in the IFA-MTP animals than in the alum animals.

**TABLE 5**

| Neutralization Titers of Immunized Baboons | | | | | | | | | | | | |
|---|---|---|---|---|---|---|---|---|---|---|---|---|
| Animal | Adjuvant^{g} | | Neutralization titers | | | | | | | | | |
| | | Virus Serum | SF2 | | | | | | | MN | | |
| | | | Bleed 5^{c} | | Bleed 12^{d} | | Bleed 22^{e} | Bleed 5^{d} | | Bleed 12^{d} | | Bleed 22^{e} |
| | | | A^{a} | B^{b} | A | B | A | A | B | A | B | B |
| 2951 | 1 | | 1,250 | nt^{f} | 400 | 160 | 500 | 90 | 140 | 800 | 128 | 250 |
| 2953 | 1 | | 500 | 50 | 475 | 8 | 2,300 | 35 | 8 | 175 | 17 | 100 |
| 2964 | 1 | | 2,100 | 10 | 2,000 | 230 | 5,000 | 100 | 80 | 350 | 210 | 600 |
| | | | | | | | | | | | | |
| 2955 | 2 | | 300 | nt | 250 | nt | 160 | 50 | 5 | 200 | 40 | 28 |
| 2965 | 2 | | 125 | nt | 350 | nt | 800 | --h | 4 | 50 | 17 | 21 |
| #positive/#animals | | | 5/5 | 2/3 | 5/5 | 3/3 | 5/5 | 4/5 | 5/5 | 5/5 | 5/5 | 5/5 |

| | | | | | | | | | | | | |
|---|---|---|---|---|---|---|---|---|---|---|---|---|
| a Assay performed by method of Steimer et al., 1988. Titers are the reciprocal of the greatest dilution to yield > 50% inhibition of p25gag production. b Assay performed by method of Nara et al., 1987. Titers are the reciprocal of the greatest dilution to yield > 90% inhibition of infectious centers. c Bleed 5 is at 10 weeks, two weeks following the third immunization. d Bleed 12 is at 23 weeks, two weeks following the fifth immunization. e Bleed 22 is at 57 weeks, two weeks following the sixth immunization. f nt = not tested. g Adjuvant 1 = IFA (incomplete Freund's Adjuvant) + 250mg MTP-PE Adjuvant 2 = Alum h -- indicates <10 for assay A; <4 for assay B | | | | | | | | | | | | |

**TABLE 6**

| Neutralization Titers of Baboons Immunized with gp120 | | | | | | | | | | | | |
|---|---|---|---|---|---|---|---|---|---|---|---|---|
| Animal | Adjuvant^{g} | Neutralization Titers | | | | | | | | | | |
| | | BRU | | | | | IIIB | | | Zr6 | | |
| | Virus Serum | Bleed 5^{d} | | Bleed 12^{e} | | Bleed 22^{f} | Bleed 5^{d} | Bleed 12^{e} | Bleed 22 | Bleed 5^{d} | Bleed 12^{e} | Bleed 22f |
| | | A^{a} | B^{b} | A | B | B | B | B | B | B | B | B |
| 2951 | 1^{e} | -C | - | 80 | - | 29 | - | 55 | 51 | - | - | - |
| 2953 | 1 | - | - | 20 | - | - | - | 15 | 6 | - | - | - |
| 2964 | 1 | - | - | 100 | 20 | 140 | 7 | 82 | 180 | - | - | - |
| 2955 | 2 | - | - | 15 | - | - | - | 15 | 11 | - | - | - |
| 2965 | 2 | - | - | - | - | - | 5 | 14 | 9 | - | - | - |
| #positive/ animals | | 0/5 | 0/5 | 4/5 | 1/5 | 2/5 | 2/5 | 5/5 | 5/5 | 0/5 | 0/5 | 0/5 |

| | | | | | | | | | | | | |
|---|---|---|---|---|---|---|---|---|---|---|---|---|
| a Assay performed by method of Steimer et al., 1988. Titers are the reciprocal of the greatest dilution to yield > 50% inhibition of p25gag production. b Assay performed by method of Nara et al., 1987. Titers are the reciprocal of the greatest dilution to yield > 90% inhibition of infectious centers. c - indicates < 10 for assay A; < 4 for assay B. d Bleed 5 is at 10 weeks, two weeks following the third immunization. e Bleed 12 is at 23 weeks, two weeks following the fifth immunization. f Bleed 22 is at 57 weeks, two weeks following sixth immunization. g Adjuvants were as described in Table 5. | | | | | | | | | | | | |

Serum collected from the highest responding gp120 -immunized baboon after six immunizations was further tested for the ability to neutralize additional virus isolates HIV-SF2, HIV-MN, HIV-RF, HIV-CC, HIV-ZR6, and HIV-NDK (Table 7a). Note that the HIV-SF2 neutralization titers were determined by the p24gag inhibition assay, while the HIV-MN neutralization was assayed by the Nara et al. infectious center protocol. Thus, the marked difference in neutralization of these two isolates can be accounted for, in part, by the two different assays used.

This data demonstrates that the gp120 protein retaining a material conformation and prepared as described above is more successful in producing cross-neutralizing antibodies than forms that do not retain natural conformation.

### Example 7

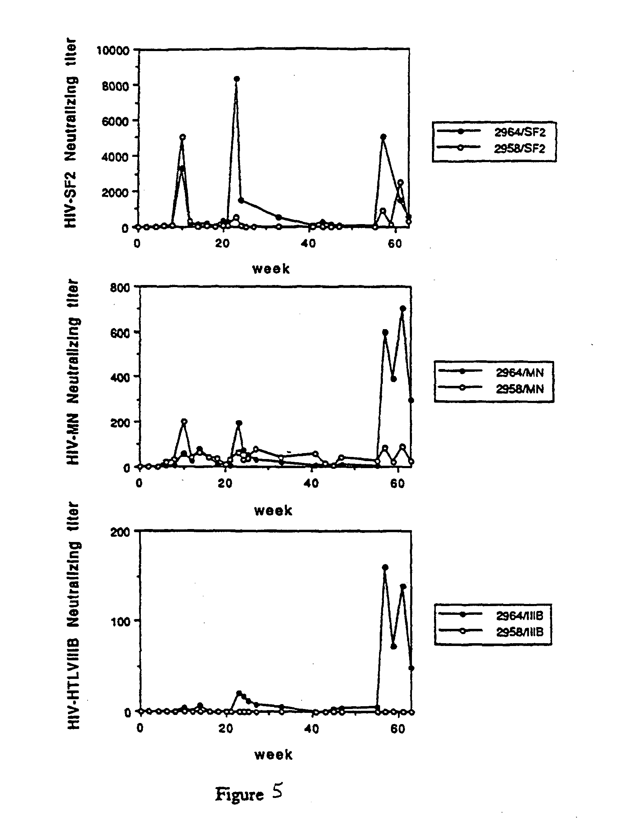

Repeated immunization of the IFA-MTP group of baboons was carried out to determine if additional repeated exposure to recombinant, native, glycosylated gp120 might result in antibodies effective in neutralizing an even broader range of isolates. Repeated immunization did not drastically alter the titers of neutralizing antibodies against HIV-SF2, HIV-MN, HIV-RF, or HIV-CC. However, repeated immunization did result in the appearance of low titer neutralizing antibodies against African isolates, HIV-ZR6 and HIV-NDK (Table 7b). The temporal development of HIV-ZR6 neutralization was examined by graphing the virus neutralization data (Figure 5) from Baboon 2964 Sera Analyzed after 0, 5, 6, 7, 8, and 9 immunizations with recombinant, native, glycosylated gp120.

Neutralization was scored by measuring the number of syncytial-forming units per ml (sfu/ml) in wells containing experimental sera (Vn, average of duplicate wells) and dividing this number by the sfu/ml virus alone (Vo, average of 8 replica wells). This fraction, Vn/Vo was plotted versus the dilution of the serum sample, and neutralization was scored by noting the dilution of serum which allowed a 90% reduction in Vn, i.e., Vn/Vo = 0.1. Samples are indicated by the key on the right, where the numbers correspond to bleeds. Bleed 0 is the prebleed, which shows no virus neutralization. Bleed 12 follows 5 immunizations; bleed 22 follows 6 immunizations; bleed 24 follows 7 immunizations; bleed 27 follows 8 immunizations; bleed 22 follows 9 immunizations.

As is evident in Figure 5, repeated boosting shifted the slope of the neutralization curve, so that neutralization was detected in bleed 32, following 9 immunizations. These results demonstrated that repeated boosting selected for antibody-producing clones that have broader specificity.

**TABLE 7a**

| HIV-VIRUS | SF2 | MN | BRU | HTLVIIIB | HXB3 | V32 | RF | CC | ZR6 | |
|---|---|---|---|---|---|---|---|---|---|---|
| TITER | 5000 | 600 | 140 | 180 | 59 | 32 | 33 | 33 | <4 | |

Results are of Bleed 22 at 57 weeks, two weeks after the sixth immunization using an assay performed by the method of Steiner et al.,

**Table 7b**

| Virus Neutralizing Titers for Baboon 2964 Following 6, 7, 8 and 9 Immunizations with gp120 | | | | | | | | |
|---|---|---|---|---|---|---|---|---|
| Neutralization Titers | | | | | | | | |
| Animal | Bleed | Immunization | HIV-SF2 | HIV-MN^{a} | HIV-RF^{b} | HIV-CC^{b} | HIV-Zr6 | HIV-NDK^{b} |
| 2964 | 22^{e} | 6 | 5,000 | 600 | 33 | 33 | --c | nt^{d} |
| 2964 | 24^{f} | 7 | 1,500 | 700 | 21 | 10 | -- | -- |
| 2964 | 27^{g} | 8 | 4,100 | 400 | 18 | 10 | -- | 5 |
| 2964 | 32^{h} | 9 | 7,000 | 310 | 18 | 6 | 4 | -- |

| | | | | | | | | |
|---|---|---|---|---|---|---|---|---|
| a Bleeds 22 and 24 were tested on different days than bleeds 27 and 32. b Bleeds 24, 27, and 32 were tested simultaneously; bleed 22 was tested on a different day. c -- indicates < 10 for assay A; < 4 for assay B. d nt = not tested. e Bleed 22 is at 57 weeks, two weeks following the sixth immunization. f Bleed 24 is at 61 weeks, two weeks following the seventh immunization. g Bleed 27 is at 67 weeks, two weeks following the eighth immunization. h Bleed 32 is at 84 weeks, two weeks following the ninth immunization. | | | | | | | | |

### Example 8

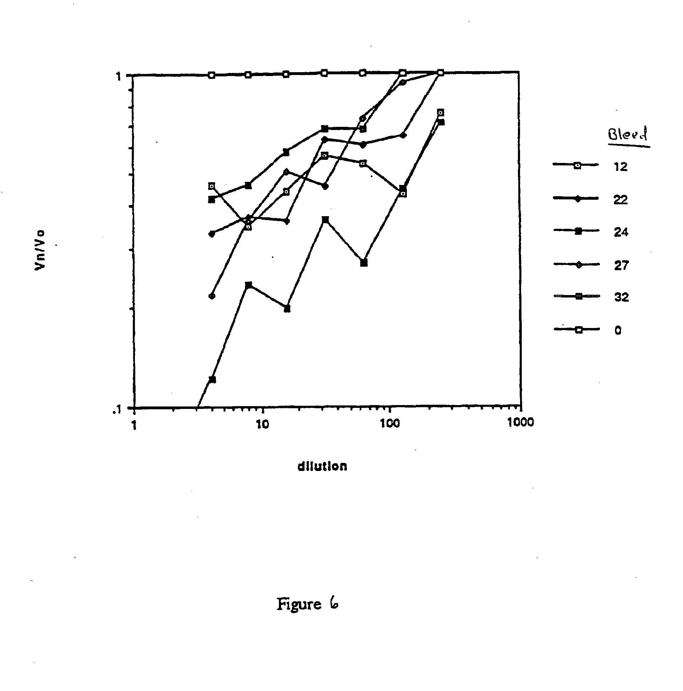

Analysis of all serum samples from two individual baboons, 2964 and 2958, further delineated differences in recombinant denatured, nonglycosylated protein and recombinant native, glycosylated (rgp120)-immunized animals (Figure 6). Baboon 2964 was vaccinated with recombinant native glycosulated protein and baboon 2958 was vaccinated with recombinant, denatured, nongly-cosulated protein. HIV-SF2 neutralization was assayed by the p25gag inhibition assay described in Haigwood et al., AIDS. Res. and Hum. Retrov. (1990) 6:855-869. All other isolates were assayed by the infectious center inhibition assay. Serum from each bleed was assayed for virus neutralization activity against HIV-SF2, HIV-MN, and HIV-HTLVIIB. Further boosting with denatured, nonglycosylated protein did not raise antibody or neutralization titers beyond the levels measured at week 10, and there was no detectable neutralization of HIV-HTLVIIIB. In the rgp120-immunized animal, HIV-SF2, HIV-MN, and HIV-HTLVIIIB titers increased following each boost, with the greatest increase observed following the rest. Patterns of neutralizing activity were similar for all three viruses, although response magnitudes differed. Emergence of HIV-HTLVIIIB neutralization was delayed relative to the other two isolates. In additional experiments in baboons discussed in Example 9 below, we have demonstrated that recombinant denatured, nonglycosylated protein formulated in MF59 was unable to induce neutralization to HIV-MN or HIV-BRU neutralizing activity (data not shown); gp120 sera neutralized these three isolates as well as HIV-ZR6 after repeated boosting (Table 8).

Figure 6 is a set of graphs showing neutralization titers of all the serum samples from baboon 2958, immunized with denatured, non-glycosylated gp120, and baboon 2964, immunized with native, glycosylated gp120. Immunization of these animals is described in Example 6.

### Example 9

Baboons were immunized with 55 mg gp120 formulated with either: microfluidized emulsion containing muramyl tripeptide-phosphatidyl ethanolamine, 100 mg (MF59); or Incomplete Freund's Adjuvant (IFA). The formulation of MF59 was 5% squalene, 0.5% Tween-80, 0.5% Span-85 with endogenous MTP-PE at 0.4 mg/ml in water, which was emulsified with a microfluidizer, and stored under argon until use. Then it was mixed with antigen by shaking and injected. Data summarizing the antigen-specific responses for the baboons are shown in Table 8.
Gp120 -specific titers also peaked, then declined, following each boost in this study. Higher titers were achieved with MF59 than with IFA. Virus neutralization was tested versus homologous and heterologous isolates was determined at weeks 10, 24, and 38, following three, four, and five immunizations respectively. The results of these assays are summarized in Table 8. In this study, animals in the MF59 group had higher titers and a greater proportion of positive animals in the group than the IFA group. Neutralizing titers effective against HIV-SF2 and HIV-MN were observed after three immunizations, and against HIV-HTLVIIIB and HIV-ZR6 after five immunizations. The animals immunized with recombinant native, glycosylated gp120 in MF59 responded with antibodies that were effective in neutralizing HIV-BRU, and HIV-ZR6 after only five immunizations. In a previous study, neutralization of African isolates was achieved only after eight (HIV-NDK) or nine (HIV-ZR6) immunizations, In addition, the titers achieved in Example 9 with recombinant native, glycosylated gp120 adjuvanted with MF59 versus HIV-ZR6 were higher. Also, the appearance of neutralizing antibodies effective against HIV-BRU and HIV-ZR6 was simultaneous in this study, in contrast to Example 6 described above. This result could be due to the adjuvant or to the regimen of immunizations, which allowed two shorter resting periods in Example 9 compared with a single long resting period in Example 6.

**TABLE 8**

| Neutralization Titers of Immunized Baboons | | | | | | | | | | | | | |
|---|---|---|---|---|---|---|---|---|---|---|---|---|---|
| Animal | Adju vant ^{h} | Neutralization Titers | | | | | | | | | | | |
| | | SF2^{a} | | | MN^{b} | | | BRU^{b} | | | Zr6^{b} | | |
| Virus Serum | | Bleed 5^{e} | Bleed 12^{f} | Bleed 14^{g} | Bleed 5^{e} | Bleed 12^{f} | Bleed 14^{g} | Bleed 5^{e} | Bleed 12^{f} | Bleed 14^{g} | Bleed 5^{e} | Bleed 12^{f} | Bleed 14^{g} |
| 7246 | 1 | nt^{c} | 70 | 110 | nt | 2 | 6 | nt | -d | 3 | - | - | - |
| 7247 | 1 | 16 | 21 | 35 | - | - | 7 | - | - | - | - | - | - |
| 7248 | 1 | 30 | 65 | 70 | 7 | 4 | 11 | - | - | 4 | - | - | - |
| 7249 | 1 | 100 | 210 | 50 | - | 18 | 25 | - | - | - | - | - | 4 |
| 7258 | 2 | 200 | 40 | 250 | - | 8 | 29 | - | - | 4 | - | - | - |
| 7259 | 2 | 30 | 60 | 70 | 15 | 45 | 29 | - | - | 4 | - | - | 8 |
| 7260 | 2 | 250 | 50 | 310 | 10 | 48 | 20 | - | - | 6 | - | - | - |
| 7261 | 2 | 100 | 31 | 350 | 22 | 51 | 20 | - | - | 5 | - | - | 6 |
| 7262 | 2 | 100 | 500 | 400 | 15 | 51 | 45 | - | - | 3 | - | - | 4 |
| #positive/ animals | | 8/8 | 9/9 | 9/9 | 5/8 | 8/9 | 9/9 | 0/8 | 0/9 | 7/9 | 0/9 | 0/9 | 4/9 |

| | | | | | | | | | | | | | |
|---|---|---|---|---|---|---|---|---|---|---|---|---|---|
| a Assay performed by method of Steimer et al., 1988. Titers are the reciprocal of the greatest dilution to yield > 50% inhibition of p25gag production. b Assay performed by method of Nara et al., 1987. Titers are the reciprocal of the greatest dilution to yield > 90% inhibition of infectious centers. c nt = not tested. d - indicates < 10 for assay a; < 4 for assay b. e Bleed 5 is at 10 weeks, two weeks following the third immunization. f Bleed 12 is at 24 weeks, two weeks following the fourth immunization. g Bleed 14 is at 38 weeks, three weeks following the fifth immunization. h Adjuvant 1 = IFA (incomplete Freund's Adjuvant) + 250mg MTP-PE Adjuvant 2 = MF59 (100mg MTP-PE) | | | | | | | | | | | | | |

### Example 10

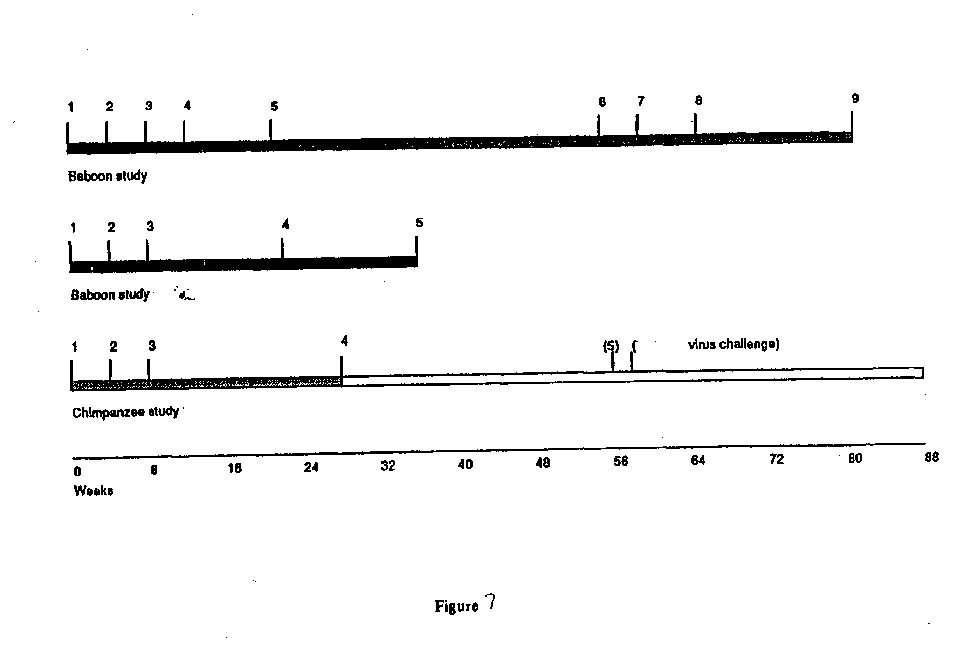

Following procedures similar to those described above for baboons, four chimpanzees (Pan troglodytes) were immunized with 55 mg gp120 adjuvanted with 2XMF59 (2 animals), adjuvant alone (1 animal), or were unimmunized (1 animal), to determine the immunogenicity of the protein in this species of primates, man's closest living relative. The experimental regimen design is set forth in Figure 7. In Figure 7, the shaded bars represent time lines (immunization schedules) for each of three studies: baboons of Example 6 (top line), baboons of Example 9 (middle line), and chimpanzees of Example 10 (bottom line). A scale of time in weeks is shown at the bottom of the figure. Immunizations are indicated by vertical bars, numbered above to indicate the immunization number, at the position on the time line of the injection. Baboons in Example 6 were immunized at weeks 0, 4, 8, 12, 21, 55, 59, 65, and 80, except baboon 2964 which was immunized at week 82 instead of week 80. Baboons in Example 9 were immunized at weeks 0, 4, 8, 22, and 36. Chimpanzees were immunized at weeks 0, 4, 8, and 28. The formulation of 2XMF59 was 10% squalene, 1% Tween-80, 1% Span-85 with endogenous MTP-PE at 0.4 mg/ml in water, which was emulsified with a microfluidizer, and stored under argon until use, when it was mixed with antigen by shaking and injected. The animals were immunized three times intramuscularly at monthly intervals, and sera have been analyzed for envelope-specific titers and for virus neutralizing antibodies for the bleeds following each immunization. The data are summarized in Table 9 for the two immunized with recombinant, native glycosylated chimpanzees. Neither of the other control chimpanzees developed gp120 -specific antibodies or neutralizing antibodies (data not shown). Both animals immunized with recombinant native glycosylated protein have developed good responses to the immunizing antigen, and both animals have virus-neutralizing antibodies effective against HIV-SF2 and HIV-MN. Serum from one of the chimpanzees also neutralized HIV-HTLVIIIB following three immunizations. The chimpanzees are boosted following a six month rest period, and sera are analyzed following this immunization. When the virus neutralizing titers against HIV-SF2 are sufficiently high, the animals are challenged with a chimpanzee-titered stock of HIV-SF2. The chimpanzees are re-immunized two weeks prior to challenge. Given the existence of neutralizing antibodies effective against heterologous isolates, the possibility for heterologous virus challenge in these same animals also exists.

**TABLE 9**

| Neutralization Titers of Chimpanzees Immunized with 2X MF-59 (gp120 Animals) | | | | | | | |
|---|---|---|---|---|---|---|---|
| Neutralization titers | | | | | | | |
| | | | Virus Isolate: | SF2 | MN | MN | IIIB |
| Animal | Bleed | Immunization | Assay: ELISA Titer | A^{a} | A | B^{b} | B |
| 10143 | 0 | 0 | <100 | --c | -- | -- | -- |
| 10143 | 1 | 1 | <100 | ntd | nt | -- | -- |
| 10143 | 2 | 2 | 5,800 | 60 | -- | -- | -- |
| 10143 | 4 | 3 | 15,700 | 280 | -- | 15 | -- |
| 10144 | 0 | 0 | <100 | -- | -- | -- | -- |
| 10144 | 1 | 1 | <100 | nt | nt | -- | -- |
| 10144 | 2 | 2 | 11,000 | 25 | -- | -- | -- |
| 10144 | 4 | 3 | 52,600 | 400 | 72 | 40 | 7 |
| | | | | | | | |
| #positive/#animals | | | 2/2 | 2/2 | 1/2 | 2/2 | 1/2 |

| | | | | | | | |
|---|---|---|---|---|---|---|---|
| a Assay A performed by the method of Steimer et al., 1988. b Assay B performed by the method of Nara et al., 1987. c -- indicates <10 for assay A; <2 for assay B. d nt = not tested. | | | | | | | |

### Deposit of Biological Materials

The following exemplary materials have been deposited on November 7, 1990, with the American Type Culture Collection (ATCC), Rockville, MD, and designated as indicated. These deposits will be maintained under the terms of the Budapest Treaty on the International Recognition of the Deposit of Micro-organisms for purposes of patent procedures.

| Deposit | ATCC # | Deposited |
|---|---|---|
| Chinese Hamster Ovary Cells | | |
| CHO-A-6a120-145-0.1-22CRL 10379 March 9, 1990 | | |
| | | |
| Chinese Hamster Ovary Cells | | |
| CHO-DG44 | CRL 10378 March 8, 1990 | |
| | | |
| E. coli | | |
| HB101 (pCMV6a120-SF2)68249 March 8, 1990 | | |

These deposits are provided merely as convenience to those of skill in the art.

## Claims

1. A process for purifying HIV glycoprotein gp120 from a medium supernatant comprising full-length non-fusion SF2 gp120, consisting of the steps of
(1) concentration of the medium supernatant using dead-end filtration and cross-flow ultrafiltration at 2-8°C;
(2) bringing the conductivity of the ultrafiltration concentrate to 1.4 mS by addition of sodium chloride;
(3) applying the concentrate to an equilibrated DEAE Sephadex A-50 ion exchange column at room temperature;
(4) bringing the unadsorbed fraction to 40% saturation in ammonium sulphate;
(5) removing precipitate by centrifugation;
(6) applying the supernatant to an equilibrated TSK Phenyl-5PW HlC column;
(7) eluting the column;
(8) bringing gp120-containing fractions to a conductivity of 165 S/cm by adding ammonium sulphate, followed by centrifugation;
(9) applying the supernatant to an equilibrated TSK Ether-5PW HlC column;
(10) eluting the column;
(11) concentration by ultrafiltration ;
(12) diafiltration against at least five volumes of 0.1 M sodium phosphate, pH 6.9.
(13) applying to a Superdex 200 gel filtration column at a total protein concentration of not more than 10 mg/ml in a volume of not more than 4% of the column volume;
(14) eluting with 0.1 M sodium phosphate, pH 6.9;
(15) concentration by ultrafiltration; and
(16) diafiltration against 5 volumes of distilled water.

## Patentansprüche

1. Verfahren zur Reinigung des HIV-Glycoproteins gp120 aus nicht fusioniertes SF2-gp120 voller Länge enthaltendem Medienüberstand, bestehend aus den Schritten:
(1) Konzentrieren des Medienüberstands unter Verwendung einer "Dead end"-Filtration und Querstrom-Ultrafiltration bei 2 bis 8°C;
(2) Einstellen der Leitfähigkeit des Ultrafiltration-Konzentrats auf 1,4 mS durch Zugabe von Natriumchlorid;
(3) Aufbringen des Konzentrats auf eine äquilibrierte DEAE-Sephadex A-50-lonenaustauschersäule bei Raumtemperatur;
(4) Einstellen der nicht adsorbierten Fraktion auf 40% Sättigung mit Ammoniumsulfat;
(5) Entfernen des Präzipitats durch Zentrifugation;
(6) Aufbringen des Überstandes auf eine äquilibrierte TSK-Phenyl-5PW-HIC-Säule;
(7) Eluieren der Säule;
(8) Einstellen der gp120 enthaltenden Fraktionen auf eine Leitfähigkeit von 165 S/cm durch Zugabe von Ammoniumsulfat, gefolgt von Zentrifugation;
(9) Aufbringen des Überstandes auf eine äquilibrierte TSK-Ether-5PW-HIC-Säule;
(10) Eluieren der Säule;
(11) Konzentration durch Ultrafiltration;
(12) Diafiltration gegen mindestens 5 Volumina 0,1 M Natriumphosphat, pH 6,9;
(13) Aufbringen auf eine Superdex-200-Gelfiltrationssäule bei einer Gesamtproteinkonzentration von nicht mehr als 10 mg/ml in einem Volumen von nicht mehr als 4% des Säulenvolumens;
(14) Eluieren mit 0,1 M Natriumphosphat, pH 6,9;
(15) Konzentrieren durch Ultrafiltration; und
(16) Diafiltration gegen 5 Volumina destilliertes Wasser.

## Revendications

1. Procédé pour la purification de la glyco-protéine gp120 de HIV à partir d'un surnageant de milieu comprenant de la gp120 non fusionnée entière, consistant en les étapes suivantes :
(1) concentration du surnageant de milieu à l'aide de filtration en cul-de-sac et d'ultrafiltration tangentielle à 2-8°C ;
(2) amener la conductivité du concentré d'ultrafiltration a 1,4 mS par addition de chlorure de sodium ;
(3) injection du concentré dans une colonne d'échange d'ions DEAE Sephadex A-50 équilibrée, à la température ambiante ;
(4) amener la fraction non adsorbée à 40 % de saturation en sulfate d'ammonium ;
(5) élimination du précipité par centrifugation ;
(6) injection du surnageant dans une colonne de TSK Phenyl-5PW HIC équilibrée ;
(7) élution de la colonne ;
(8) amener les fractions contenant gp-120 à une conductivité de 165 S/cm par addition de sulfate d'ammonium, suivie d'une centrifugation ;
(9) injection du surnageant dans une colonne TSK Ether-5PW HIC équilibrée ;
(10) élution de la colonne ;
(11) concentration par ultrafiltration ;
(12)diafiltration contre au moins cinq volumes de phosphate de sodium 0,1 M, pH 6,9 ;
(13) injection dans une colonne de filtration sur gel Superdex 200, a une concentration totale de protéine d'au maximum 10 mg/ml dans un volume d'au maximum 4 % du volume de la colonne ;
(14) élution avec du phosphate de sodium 0,1 M, pH 6,9 ;
(15) concentration par ultrafiltration ; et
(16) diafiltration contre 5 volumes d'eau distillée.
